# EUROPEAN PATENT APPLICATION

(11) **EP 4 578 471 A2**
(43) Date of publication of application: **02.07.2025**
(21) Application number: 25171508.2
(22) Date of filing: 11.03.2019
(51) Int. Cl.: A61L 27/50

(54) **A METHOD OF PROVIDING FEATURES ON AN IMPLANTABLE MATERIAL INVOLVING THE USE OF LASER, IMPLANTABLE VASCULAR PROSTHESES AND IMPLANTABLE MATERIALS PROCESSED ACCORDING TO SAID METHOD**

(62) Divisional of application: 19716981.6
(71) Applicant: Corcym S.r.l., 20141 Milano (MI) (IT)
(72) Inventor: STRASLY, Marina, 20141 Milano (MI) (IT); GUALA, Carlo, 20141 Milano (MI) (IT); VALLE, Francesco, 20141 Milano (MI) (IT)
(74) Representative: Buzzi, Notaro & Antonielli d'Oulx S.p.A.

(57) **Abstract**

Embodiments herein relate to a method of providing features (LCD, HD, HD_IN, HD_OUT) on an implantable material (ID), the method comprising:
- providing an implantable material (ID) comprising a decellularized sterilized mammalian tissue having an extracellular matrix, wherein a plurality of interstitial spaces of the extracellular matrix include a solution of one or more polyols,
- providing one or more features (LCD, HD, HD_IN, HD_OUT) on said implantable material (ID) by laser treatment.

## Description

### Technical field

The present disclosure relates to implantable materials, particularly decellularized mammalian tissue having an extracellular matrix, and implantable cardiovascular prostheses featuring at least one portion made of such implantable materials.

### Background

Biological prostheses are medical devices that use mammalian donor tissues. Examples of suitable mammalian tissues include, for example, bovine, porcine, ovine, equine, and human tissues. Depending on the various medical uses of a biological prosthesis, the donor tissue may include, for example, cardiac valves, pericardium, tendons, ligaments, dura mater, skin, and veins.

Many processes and chemicals employed to treat tissue for a bioprosthesis result in crosslinking of the extracellular collagen matrix within the tissue. Crosslinking or "fixing" of the extracellular matrix improves the strength and biological durability of the tissue. Aldehyde based processes are a typical example of this approach. Aldehyde based processes are also used to provide chemical sterilization to the bioprosthesis. Applied to a decellularized tissue or to a material obtained via tissue engineering, these crosslinking/sterilization methods reduce the ability of the tissue to remodel. In some applications, crosslinking or "fixing" of the extracellular matrix improves the strength and biological durability of the tissue.

As an additional drawback, aldehyde based fixing processes result in a treated implantable material requiring specific storage solutions to preserve the integrity thereof, such as for example, storage in bacteriostatic liquid storage solution. This is commonly referred to as a "wet" implantable material.

Another group of implantable materials include decellularized, fixed mammalian tissue that is sterilized and preserved sterile and dry without impairing the ability of the extracellular matrix to be remodeled, or else implantable materials including decellularized mammalian tissue that has been fixed.

In either case, the decellularized tissue may be made by removing the cells from mammalian tissue, such as a porcine heart valve, leaving behind the extracellular matrix. Other mammalian tissues may include bovine pericardium, equine pericardium or other porcine tissues such as the intestinal mucosa. Alternatively, the tissue may be made by seeding a polymeric mold with mammalian cells which grow along the polymeric mold to produce the extracellular matrix of collagen or of other structural proteins. Once completed, the cells are removed, leaving behind the extracellular matrix.

After decellularization, the decellularized tissue (or polymer with attached extracellular matrix, in alternative embodiments) is treated with a solution in order to reduce the retained water in the tissue. In some embodiments, the treatment solution includes one or more polyols, for example a mixture of glycerol and one or more polyols other than glycerol. In some embodiments, the one or more polyols can include at least one of 1,2 propanediol, 1,2 octanediol, 1,4 butanediol, fructose, xylitol, mannitol, sorbitol, lactulose, lactic acid, maltose, glucose, galactose, erythritol, lactobionic acid or its salts, and hyaluronic acid of various molecular weights and crosslinked to various degrees or not crosslinked.

Implantable materials of these latter types are commonly referred to as "dry" implantable materials. An advantage thereof is the possibility of long-time dry storage with no significant deterioration of the material or the properties thereof. In other words, such implantable materials can be stored without soaking the same into a preservation solution as it happens with the "wet" implantable materials.

Whether "wet" or "dry", the available implantable materials may be used for the manufacturing of cardiovascular prosthesis such as heart valve prostheses or prosthetic conduits. The manufacturing of these prostheses involves the provision of features on a starting implantable material, which generally comes as a sheet material. These features may include for example - without this being an exhaustive list - the perimeter of a functional portion of a prosthetic heart valve such as a valve leaflet, or a leaflet edge, or else a through hole providing a sewing location for assembly of the prosthesis and/or assembly of the prosthesis onto a supporting structure. For a prosthetic vascular conduit, such features may include once again through holes as sewing references or opening a port in a conduit wall for subsequent fitting of a branching portion, such as in vascular prostheses for bifurcated portions of the vasculature.

Under these circumstances, the manufacturing of cardiovascular prostheses (e.g. prosthetic conduits, prosthetic valved conduits, and heart valve prostheses) with the biological implantable materials discussed in the foregoing is generally subject to a tradeoff between dimensional consistency and repeatability of the products, and preservation of the performances of the implantable material.

Processing of the implantable material to provide features thereto may be done via purely mechanical processing (e.g. mechanical cutting) or via optoelectronic processing, including e.g. laser treatment. Both these techniques are affected by certain drawbacks. Mechanical cutting may in some instances require extensive manipulation of the implantable material just to make the interaction with the cutting tools happen. That extensive manipulation may lead to premature degradation of the material, well ahead of any implantation event. Additionally, certain features may hardly - if at all - be provided on the implantable material due to dimensional constraints. For example, the provision of sewing holes or complex contours by way of mechanical processing is generally out of reach as that would weaken the material or damage it to the point of rendering the same unfit for implantation.

As to laser processing, this is generally limited to thermal-laser cutting via a CO₂ laser. While laser cutting or treatment allows higher precision and can negotiate complex cutting profiles, the main drawback that comes with it - which as a matter of fact limits both the diffusion and the effectiveness of laser treated implantable material - is the interaction of laser beams with water or water-based components embedded in the implantable material. Especially with "wet" implantable materials, which today are the main choice for cardiovascular prostheses due to consolidated storage protocols, thermal power transferred to the implantable material during, e.g., laser cutting, causes sudden, explosive vaporization of water or liquid fractions, thus producing very irregular and/or burnt out cut edges.

When it comes to cutting through holes for, e.g., sewing threads, the above interaction results in holes cut with a largely irregular shape, and with burned out edges all around. Any such hole is completely unsuitable for implantation as it is extremely prone to fail when subject to tension from tightening the stitching threads. Even if those were to resist static load from stitching threads, they would suffer from poor fatigue resistance, thus leading to a major failure of, e.g., a prosthetic valve leaflet when subject to repeated blood pumping cycles.

### Summary

In a first example, a method of providing features on an implantable material, the method comprising:
- providing an implantable material comprising a decellularized sterilized mammalian tissue having an extracellular matrix, wherein a plurality of interstitial spaces of the extracellular matrix include a solution of one or more polyols,
- providing one or more features on said implantable material by laser treatment.

In a second example according to the first example, wherein said laser treatment includes laser cutting.

In a third example according to the first or the second examples, wherein said features include geometry features.

In a fourth example according to the first or the second examples, wherein said laser treatment includes CO2 laser treatment.

In a fifth example the first or the second examples, wherein said laser treatment includes femtosecond laser treatment.

In a sixth example according to any of examples second to fifth, wherein said providing one or more features on said pericardium tissue by laser treatment includes laser cutting a line feature

In a seventh example according to the sixth example, wherein said laser cutting a line feature includes laser cutting a perimeter and/or an edge of an element out of said implantable material.

In an eighth example according to the sixth example, wherein said laser cutting a line feature includes laser cutting a closed loop feature.

In a ninth example according to the eighth example, wherein said laser cutting a closed loop feature includes laser cutting one or more through holes.

In a tenth example according to any of examples sixth to ninth, wherein said laser cutting a line feature is provided by a CO₂ laser having a wavelength comprised between 9000 nm and 12000 nm, a maximum rated power comprised between 15 and 40 W, a maximum rated processing velocity comprised between 1500 mm/s and 2300 mm/s, and a lens focal length comprised between 10 and 50 mm.

In an eleventh example according to the tenth example, laser cutting a perimeter or an edge by a CO₂ laser is provided according to the following parameters
i) Power: 40% to 60% of maximum rated power
ii) Velocity: 2% to 10% of maximum rated processing velocity
iii) Frequency: 1 kHz to 10 kHz
iv) Number of passes: 1 to 10
v) Lens focal length: 25,4 mm

In a twelfth example according to the tenth example, laser cutting one or more through holes by a CO₂ laser is provided according to the following parameters
i) Power: 40% to 50% of maximum rated power
ii) Velocity: 0.5% to 2% of maximum rated processing velocity
iii) Frequency: 1 kHz to 10 kHz
iv) Number of passes: 1
v) Lens focal length: 25,4 mm.

In a thirteenth example according to any of examples sixth to ninth, wherein said laser cutting a line feature is provided by a femtosecond laser having a pulse duration of 400 femtoseconds (fs) or less, a wavelength comprised between 400 nm and 600 nm, and a lens focal length comprised between 90 mm and 110 mm.

In a fourteenth example according to the thirteenth example, wherein laser cutting a perimeter or an edge by a femtosecond laser is provided according to the following parameters:
i) Power: 4W to 6W
ii) Velocity: 1000 mm/s to 2000 mm/s
iii) Frequency: 80 kHz to 100 kHz
iv) Number of passes: 60 to 100
v) Lens focal length: 100 mm.

In a fifteenth example according to the thirteenth example, wherein laser cutting one or more through holes by a femtosecond laser is provided according to the following parameters:
i) Power: 4W to 6W
ii) Velocity: 1000 mm/s to 2000 mm/s
iii) Frequency: 80 kHz to 100 kHz
iv) Number of passes: 80 to 180
v) Lens focal length: 100 mm.

In a sixteenth example according to any of examples second to fifth, wherein said providing one or more features on said implantable material by laser treatment includes laser ablation to provide a sculpturing of said implantable material.

In a seventeenth example according to the sixteenth example, wherein said laser ablation is provided by a CO₂ laser having a wavelength comprised between 9000 nm and 12000 nm, a maximum rated power comprised between 15 and 40 W, a maximum rated processing velocity comprised between 1500 mm/s and 2300 mm/s, and a lens focal length comprised between 10 mm to 50 mm.

In an eighteenth example according to the seventeenth example, wherein said laser ablation is provided according to the following parameters
i) Power: 20% to 70% of maximum rated power
ii) Velocity: 20% to 65% of maximum rated processing velocity
iii) Frequency: 300 ppi to 1000 ppi (points per inch)
iv) Number of passes: 1 to 10
v) Lens focal length: 25,4 mm.

In a nineteenth example according to the sixteenth example, wherein said laser ablation is provided by a femtosecond laser having a pulse duration of 400 femtoseconds (fs) or less, a wavelength comprised between 400 nm and 600 nm, and a lens focal length comprised between 90 mm and 110 mm.

In a twentieth example according to the nineteenth example, wherein said laser ablation is provided according to the following parameters:
i) Power: 2W to 6W
ii) Velocity: 7000 mm/s to 9000 mm/s
iii) Frequency: 200 kHz to 500 kHz
iv) Number of passes: 10 to 50
v) Lens focal length: 100 mm.

In a twenty-first example according to the first example, wherein the implantable material is dried and sterilized.

In a twenty-second example according to the twenty-first example, wherein the solution of one or more polyols includes one or more polyols other than glycerol, and glycerol.

In an twenty-third example according to the twenty-second example, wherein the glycerol is present in an amount of about 1 vol. % to about 50 vol. % of the mixture and the one or more polyols other than glycerol are present in an amount of about 50 vol. % to about 99 vol. % of the mixture.

In a twenty-fourth example according to the twenty-second example, wherein the one or more polyols other than glycerol includes at least one of 1,2 propanediol, 1,2 octanediol, 1,4 butanediol, fructose, xylitol, mannitol, sorbitol, lactulose, lactic acid, maltose, glucose, galactose, erythritol, lactobionic acid or its salts, and hyaluronic acid.

In a twenty-fifth example according to the twenty-second example, wherein the one or more polyols other than glycerol are selected from the group consisting of 1,2 propanediol, 1,4 butanediol, lactobionic acid and its salts, and combinations thereof.

In a twenty-sixth example according to the twenty-second example, wherein the one or more polyols other than glycerol consists essentially of 1,2 propanediol.

In a twenty-seventh example according to any of the previous examples, wherein said implantable material includes bovine pericardium.

In a twenty-eighth example according to the twenty-first example, wherein said bovine pericardium has a thickness comprised between 0,2 mm and 0,7 mm.

In a twenty-ninth example, an implantable cardiovascular prosthesis having at least a portion made of an implantable material having features provided by the method according to any of examples first to twenty-eighth.

In a thirtieth example according to the twenty-ninth example, wherein the prosthesis is a heart valve prosthesis including a plurality of prosthetic valve leaflets made of said implantable material.

In a thirty-first example according to the thirtieth example, wherein each leaflet features laser cut edges, and one or more laser cut through holes providing sewing locations.

In a thirty-second example according to the thirty-first example, wherein the leaflets are sewn to a supporting structure via sewing threads routed through said sewing holes and said supporting structure.

In a thirty-third example according to the thirty-second example, wherein said supporting structure is radially contractible.

In a thirty-fourth example according to any of examples thirtieth to thirty-third, wherein said supporting structure is an armature comprising:
an annular part
a pattern of arched struts carried by said annular part, said pattern of arched struts having proximal ends connected to said annular part, and distal ends spaced axially from the proximal ends and opposite said annular part,
a plurality of sets of anchoring formations configured to protrude radially outwardly of said annular part, each set being supported by at least one of said annular part and a corresponding arched strut, and
a plurality of support posts, each support post being supported by adjacent arched struts,
wherein the sets of anchoring formations alternate with the support posts around said longitudinal axis.

In a thirty-fifth example according to any of examples thirtieth to thirty-fourth, wherein the prosthetic leaflets (4) include sculptured features, said sculptured features including at least one of:
- a contoured sculptured pattern,
- a deformation pattern
- a layer ablation.

In a thirty-sixth embodiment, a method of making an implantable cardiovascular prosthesis according to any of embodiments twenty-ninth to thirty-fifth, the method including:
- providing a sheet of said implantable material comprising a decellularized sterilized mammalian tissue having an extracellular matrix, wherein a plurality of interstitial spaces of the extracellular matrix include a solution of one or more polyols,
- laser cutting a plurality of through line features into said implantable material to define corresponding perimeters of a plurality of leaflets,
- laser cutting a plurality of through closed loop features into said implantable material to provide a plurality of sewing holes for each leaflet,
- sewing the leaflets into a valvular sleeve in correspondence of said sewing holes,
- sewing the leaflets to a supporting structure of said implantable cardiovascular prosthesis.

In a thirty-seventh embodiment according to the thirty-sixth embodiment, further including sewing the leaflets into a valvular sleeve in correspondence of said sewing holes prior to sewing the leaflets to the supporting structure.

In a thirty-eight embodiment according to the thirty-sixth or the thirty-seventh embodiment, wherein sewing the leaflets to a supporting structure of said implantable cardiovascular prosthesis includes sewing the leaflets to the support posts of said supporting structure.

In a thirty-ninth embodiment according to embodiments thirty-sixth to thirty-eighth, further including laser cutting a strip of said implantable material, and sewing it to said annular part of said supporting structure.

In a fortieth embodiment according to embodiments thirty-sixth to thirty-ninth, further including providing a sculpturing by laser ablation within the perimeter of each leaflet.

In a forty-first embodiment, a kit comprising a delivery instrument and an implantable cardiovascular prosthesis according to any of embodiments twenty-ninth to thirty-fifth.

In a forty-second embodiment, the kit according to the forty-first embodiment, wherein the delivery instrument includes a carrier portion, and wherein the implantable cardiovascular prosthesis is pre-loaded onto the carrier portion with at least one portion thereof in a radially collapsed condition.

In a forty third embodiment according to the forty-second embodiment, wherein the carrier portion includes a first deployment element and a second deployment element, each of the deployment elements being configured to hold a respective portion of the implantable cardiovascular prosthesis in a radially collapsed condition.

In a forty-fourth embodiment according to the forty-third embodiment, wherein the implantable cardiovascular prosthesis is a heart valve prosthesis according to the thirty-fourth embodiment, and wherein:
an inflow portion of the heart valve prosthesis is associated to said first deployment element,
an outflow portion of the heart valve prosthesis is associated to said second deployment element and is held in a radially collapsed condition by said second deployment element,
the anchoring formations are left radially unconstrained by the first deployment element and the second deployment element.
In a forty-fifth embodiment according to the forty-fourth embodiment, wherein said inflow portion is held in a radially collapsed condition by said first deployment element.

### Brief description of the figures

Further features and advantages will become apparent from the following description with reference to the annexed figures, given purely by way of non limiting example, wherein:
- Figures 1 to 14 relate to a method according to embodiments wherein CO₂ laser is used to treat an implantable material, and furthermore wherein odd-numbered figures (1, 3, 5, 7, 9, 11, 13) refer to a comparative example obtained in accordance with prior art guidelines and techniques, while even-numbered figures (2, 4, 6, 8, 10, 12, 14) refer to embodiments obtained in accordance with the disclosure, and
- Figures 15 to 18 relate to a method according to embodiments wherein a femtosecond laser is used to treat an implantable material, and furthermore wherein odd-numbered figures (15, 17) refer to a comparative example obtained in accordance with prior art guidelines and techniques, while even-numbered figures (16, 18) refer to embodiments obtained in accordance with the disclosure.

- figures 19A, 19B, 19C and 20 to 24 are exemplary of a cardiovascular prosthesis including elements or components made according to embodiments of the method herein,
- figures 25 and 26 are exemplary of features provided on components of a cardiovascular prosthesis in accordance with embodiments of the method herein,
- figures 28, 30 are exemplary of non-through features provided by the method according to the invention, while figures 27 and 29 are exemplary of the same features provided in accordance with the prior art, and
- figures 31, 32 are exemplary of a kit according to embodiments.

### Detailed description

Various embodiments of a method according to the invention are shown herein in relation to the processing of an implantable material to provide a feature thereto. According to the disclosure, the term "feature" may identify any geometry feature to be provided onto the starting implantable material to adapt the same to finished product use, such as for example in the manufacturing of cardiovascular prostheses including heart valve prostheses and conduit prostheses.

Geometry features may include both shape geometry defining features such as for example a perimeter or an edge of an element to be cut out of the implantable material, and functional geometry features such as, for instance, through holes to provide sewing locations or else through openings having an elongated shape to receive a stent support post, or - yet further - a surface sculpturing or scoring involving non-through features (e.g. ablated portions having a depth lower than a thickness of the implantable material) which may be used, for instance, to bestow specific deformability patterns to an element of the to-be cardiovascular prosthesis subsequently manufactured using the implantable material treated by the method according to the disclosure.

In embodiments, the features are provided by laser treatment. In embodiments, such laser treatment includes laser cutting. In other embodiments, such laser treatment includes laser ablation to provide non-through features.

Laser treatment according to embodiments may be provided as CO₂ laser treatment or cutting. According to other embodiments, laser treatment may be provided as femtosecond laser treatment or cutting.

When laser treatment to provide features on the implantable material, whether executed by CO₂ laser or femtosecond laser, includes laser cutting, the one or more features on the implantable material include laser cutting a line feature.

An example of laser cutting a line feature includes laser cutting a perimeter and/or an edge of an element out of said implantable material. A line feature may be a straight line feature, or a line developing according to a curved profile, a spline, a broken line, etc.

For example, laser cutting line feature may include laser cutting the perimeter of a valve leaflet out of a patch of implantable material or laser cutting an outflow end edge of the same leaflet to achieve a neater profile to reduce blood deposits and/or blood turbulence. Alternatively, laser cutting a line feature may include laser cutting the outline of lumen-type cardiovascular prosthesis such as a prosthetic conduit or a portion thereof.

Laser cutting a line feature may also include laser cutting a closed loop feature. Closed loop features may include, for example, holes or eyelets or generally features leaning more towards the functional side. An example of this includes laser cutting through holes for routing sewing threads of a to-be cardiovascular prosthesis. For example, the method according to embodiments may be used to provide sewing holes for mutual attachment of adjacent leaflets of a bi- or tri-cuspid prosthetic heart valve, and/or for attachment of a valvular sleeve including a plurality of leaflets to a supporting structure or armature including, for instance a perimeter frame or a radially contractible armature such as a stent.

The method disclosed herein involves the use of an implantable material comprising a decellularized sterilized mammalian tissue having an extracellular matrix, wherein a plurality of interstitial spaces of the extracellular matrix include a solution of one or more polyols. In some embodiments, said one or more polyols include at least glycerol. In some embodiments, the solution of one or more polyols includes one or more polyols other than glycerol, and glycerol.

In one embodiment, the glycerol is present in an amount of about 1 vol. % to about 50 vol. % of the mixture and the one or more polyols other than glycerol are present in an amount of about 50 vol. % to about 99 vol. % of the mixture.

In another embodiment, the one or more polyols other than glycerol includes at least one of 1,2 propanediol, 1,2 octanediol, 1,4 butanediol, fructose, xylitol, mannitol, sorbitol, lactulose, lactic acid, maltose, glucose, galactose, erythritol, lactobionic acid or its salts, and hyaluronic acid.

In yet another embodiment, the one or more polyols other than glycerol are selected from the group consisting of 1,2 propanediol, 1,4 butanediol, lactobionic acid and its salts, and combinations thereof.

In yet another embodiment, the one or more polyols other than glycerol consists essentially of 1,2 propanediol.

In embodiments, the treatment with the solution includes immersing the tissue in the treatment solution at a temperature ranging from about 5°C to about 35°C for a time ranging from about 2 hours to about 100 hours. In other embodiments, treatment with the solution includes repeated immersions two to three times, each immersion time ranging from about 1 hour to about 20 hours. In yet other embodiments, the treatment with the solution may include spreading the treatment solution on the tissue or spraying it on the tissue.

In embodiments, during the treatment with the solution, the tissue and the treatment solution can be held under pressure greater than about 100 kPa to enhance movement of the treatment solution into the tissue. In embodiments, the pressure under which the tissue and the treatment solution are held during the treatment can range from about 200 kPa to about 10,000 kPa. In embodiments, the pressure under which the tissue and the treatment solution are held during the treatment can range from about 300 kPa to about 1,000 kPa.

In embodiments, this applying to each and every single embodiment of those mentioned above, the implantable material is dried, to essentially remove aqueous components therein, and sterilized.

The treated tissue may be completely dried by exposing it to air for a time ranging from about 2 hours to about 96 hours at a temperature ranging from about 20°C to about 45°C. In some embodiments, the treated tissue of the implantable material may be dried to remove substantially all of the water, so that the implantable material is substantially free of water. The treatment solution of one or more polyols and, optionally, glycerol, remains within the interstitial spaces of the extracellular matrix, and preserves the integrity and flexibility of the implantable material without the need of storing it in a water based storage solution.

In embodiments, sterilizing the dried tissue may include exposing the dried tissue to ethylene oxide gas, avoiding temperatures above 50°C in order not to damage the tissue itself. In embodiments, sterilizing the dried tissue may include exposing the dried tissue to ionizing radiation, such as gamma radiation. In embodiments, sterilizing the dried tissue may include exposing the dried tissue to UV light. In some embodiments, the dried tissue may be sterilized by exposure to any combination of ethylene oxide gas, ionizing radiation, and UV light. None of these sterilization methods induces tissue fixation, so remodeling is not impaired.

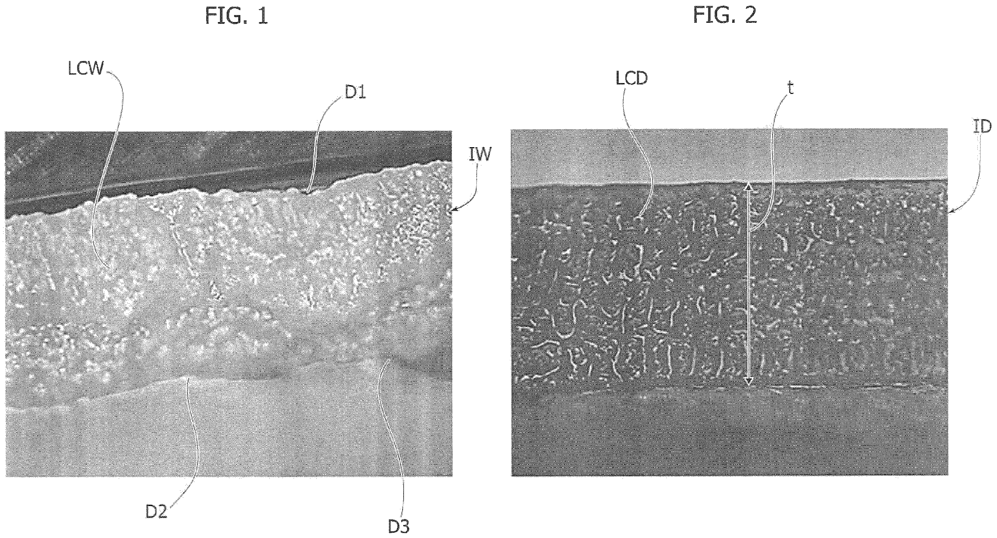

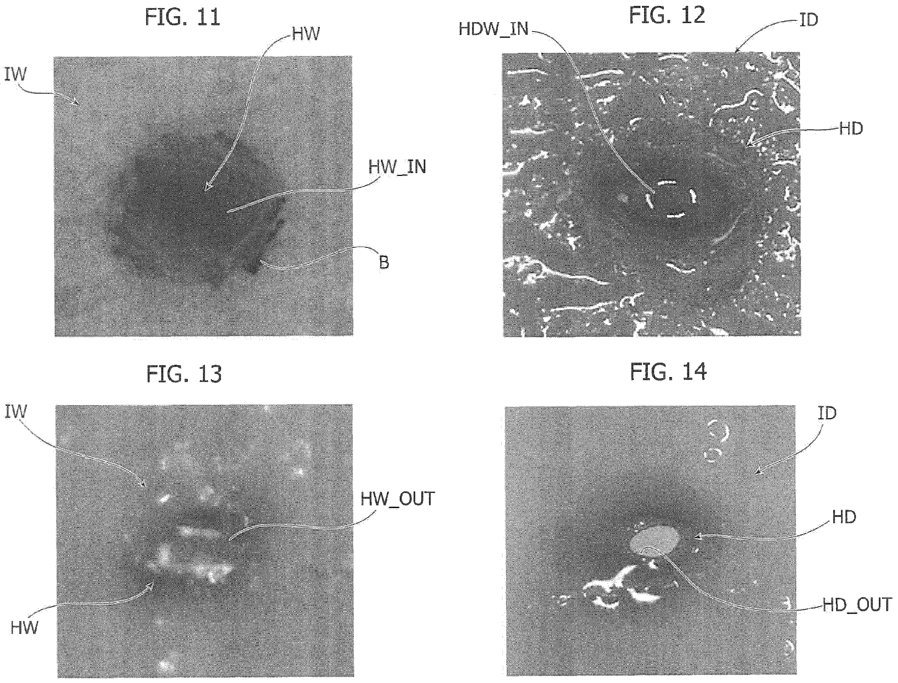

In embodiments, the implantable material includes bovine glutaraldehyde fixed pericardium. Figures 1 to 18 attached to the disclosure all feature examples or embodiments involving the use of bovine pericardium. Bovine pericardium may have a thickness comprised between 0,2 mm and 0,7 mm.

Figure 2 is representative of embodiments of the method wherein the implantable material is decellularized bovine pericardium having an extracellular matrix, wherein plurality of interstitial spaces is filled with 50% wt glycerol + 50% wt 1,2 propandiol and then dried by mechanically removing (e.g. by rolling, using a spreader, using a rolling pin) the excess filler. The bovine pericardium may have a thickness t of 0,4 mm, and generally comprised between 0,2 mm and 0,7 mm. The pericardium is dried and sterilized, and as such it may be identified as "dry" pericardium.

Figure 2 is representative of embodiments wherein the feature provided onto the implantable material is a line feature, particularly a linear (straight line) cut. The straight cut is provided by means of a CO₂ laser. Figure 1 is representative of the same operating conditions, but involves the use of "wet" bovine pericardium as implantable material, according to the prior art. The "wet" implantable material of figure 1 is identified by reference IW, while the "dry" implantable material of figure 2 is identified by the reference "ID". Accordingly, the linear cut is identified by references LCW for the "wet" material and "LCD" for the "dry" material.

The CO₂ laser used with the embodiment of Figure 2, and the example of Figure 1, has a wavelength of 10600 nm, wherein the wavelength may in general be comprised between 9000 nm and 12000 nm, a maximum rated power of 30 W, wherein maximum rated power may in general be comprised between 15 and 40 W, a maximum rated processing velocity of 1800 mm/s, wherein the maximum rated processing velocity may in general be comprised between 1500 mm/s and 2300 mm/s, and a lens focal length of 25,4 mm, wherein the lens focal length may in general be comprised between 10 mm and 50 mm.

Operating parameters applying to the embodiment of figure 2 correspond to the following
i) Power: 40% to 60% of maximum rated power
ii) Velocity: 2% to 10% of maximum rated processing velocity
iii) Frequency: 1 kHz to 10 kHz
iv) Number of passes: 1 to 10
v) Lens focal length: 25,4 mm.

As visible in figure 2 when compared to figure 1, the surface exposed following cutting exhibits markedly more regular structure, pattern and layout than those exhibited by the exposed surface of figure 1. Figure 1 provides experimental evidence of the circumstances put forward in the introductory portion of this disclosure relating to prior art methods and techniques involving the use of "wet" pericardium as implantable material. Thermal power from laser beams is transferred to the areas surrounding the cutting front, with little heat transfer to neighboring areas due to the generally low thermal conductivity of biological materials. Accordingly, heat is dissipated primarily at the areas surrounding the cutting front, which feature a high liquid concentration due to the water or - generally-the aqueous compounds that soak the interstitial space of the extracellular matrix of the decellularized pericardium. As heat transfers to water or aqueous compounds at the cutting front, the same experience a sudden vaporization featuring extremely high vaporization rates. In other words, the water or aqueous compounds exhibits a quasi-explosive behavior when the implantable material is being impinged upon and cut by the laser beams.

Quasi-explosive vaporization brings about two undesired effects, namely:
a) the surrounding areas undergo surface modifications featuring geometric singularities such as distortions, buckling, indentations or the like as a result of the impulsive expansion of liquid, with subsequent impulsive compression of the surrounding areas; the upper and lower edge may exhibit amplified dents or pits due to the higher deformability thereof, as visible in figure 1 - see dents D1, D2, D3.
b) although this effect may be increasingly mitigated as the thickness of the material and/or the length of the linear cut increase, the sudden vaporization of water or aqueous compounds removes the sole effective heat-transferring material at the cutting front; as a result, the heat flow not gone into vaporization of water or aqueous compounds impinges upon the biological tissue at the cutting front, which is not capable of significant heat transfer. Accordingly, the heat flow pattern experiences a peak in correspondence of the tissue at the cutting front. In this case, the mitigation effect is visible in that no burnout areas are visible on the cutting surface of the pericardium material, but the material may nevertheless be affected, i.e., as far as fatigue resistance performances of the element or component cut out of the implantable material are concerned.

No such effects are observed in figure 2: the thickness t remains substantially uniform across the length of the linear cut LCW, which may be regarded as bearing witness to the substantial absence of defects under item a) above, and the same thickness t shows no signs whatsoever of burnouts or the like. This is due to the filling of the extracellular matrix with glycerol. Glycerol is around 25% more dense than water, and at room temperature it is more viscous as well. When the "dry" implantable material ID is impinged upon by laser beams for providing the linear cut LCD, the heat flow towards the biological tissue is absorbed almost entirely by glycerol. Being it more dense and viscous than water, and specifically more viscous, sudden and violent vaporization phenomena are substantially ruled out: glycerol provides a thermal sink that absorbs the local thermal shock generated by laser cutting process.

Accordingly, the features of the processed element or component are not significantly different - if at all - from those of the starting material, and fatigue performances are not affected either.

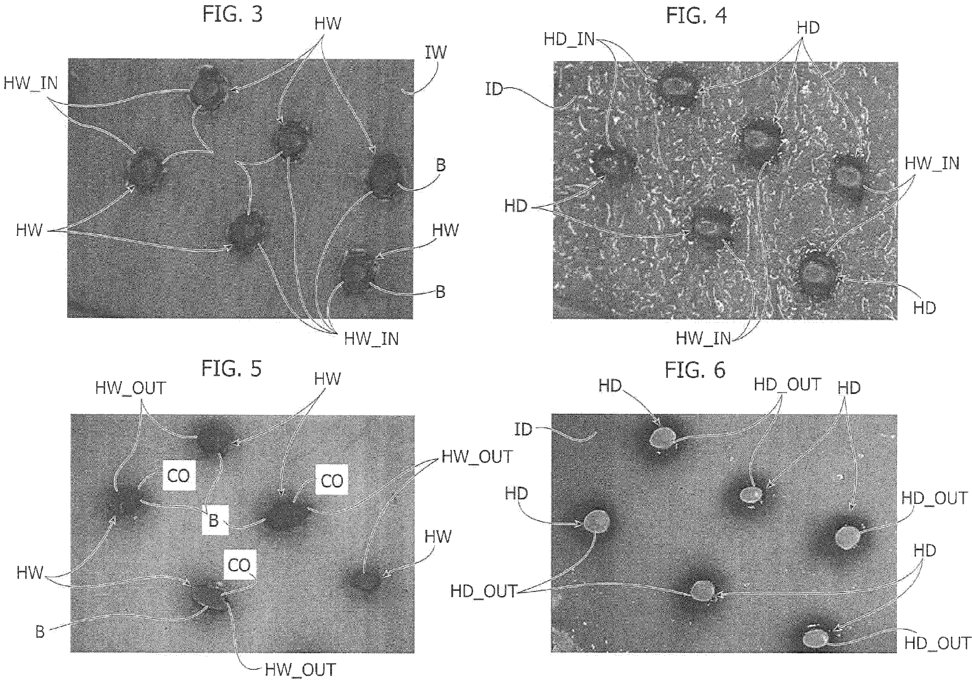

Figures 4, 6 are representative of embodiments wherein the feature provided onto the implantable material is a closed loop feature, particularly the cutting of a through hole to provide a sewing reference. The through hole is cut by means of a CO₂ laser.

Figures 3, 5 are representative of the same operating conditions, but - similar to figure 1 - they involve the use of "wet" bovine pericardium as implantable material, according to the prior art. The "wet" implantable material of figures 3, 5 is again identified by reference IW, while the "dry" implantable material of figures 4, 6 is again identified by the reference "ID". Accordingly, the through holes are identified by references HW for the "wet" material and "HD" for the "dry" material. Figure pairs 3, 5 and 4, 6 show flip sides of the same implantable material sample wherein the through holes HW and HD respectively have been provided.

Accordingly, whether made on a wet or dry material, the holes appearing in the figures are also identified based on the visible rim being the inlet end or the outlet end of the laser beam. Inlet ends are identified by reference HW_IN for the "wet" material, and by reference HD_IN for the "dry" material. Consistently, outlet ends are identified by reference HW_OUT for the "wet" material, and by reference HD_OUT for the "dry" material.

The CO₂ laser used with the embodiment of Figures 4, 6, and the example of Figures 3, 5, is the same as that used with the linear cuts LCW and LCD of figures 1 and 2, i.e. it has a wavelength of 10600 nm, wherein the wavelength may in general be comprised between 9000 nm and 12000 nm, a maximum rated power of 30 W, wherein maximum rated power may in general be comprised between 15 and 40 W, a maximum rated processing velocity of 1800 mm/s, wherein the maximum rated processing velocity may in general be comprised between 1500 mm/s and 2300 mm/s, and a lens focal length of 25,4 mm, wherein the lens focal length may in general be comprised between 10 mm and 50 mm.

Operating parameters applying to the embodiment of figures 4, 6 (as well as to the example of figures 3, 5) are however different from those used on the implantable materials IW and ID of figures 1, 2, and are chosen with a view of the specific feature being provided, i.e. a through hole.

This being said, the parameters correspond to the following
i) Power: 40% to 50% of maximum rated power
ii) Velocity: 0.5% to 2% of maximum rated processing velocity
iii) Frequency: 1 kHz to 10 kHz
iv) Number of passes: 1

As witnessed by the number of passes, in embodiments the through hole may be cut by focusing a laser beam on a spot/an area on the implantable material at the location where the theoretical axis of the hole is intended to be positioned.

Additionally, with reference to the operational ranges posted above, the implantable material patches shown in figures 3, 5 and 4, 6, are processed according to the upper bands of each range.

With reference to figures 3, 5, the drawbacks from using a "wet" implantable material are amplified when cutting closed loop geometries, especially a through hole. With closed loop geometries, the heat flow remains confined around the focus spot/area and around the hole. This has two immediate consequences, namely:
i) distortion effects due to sudden and explosive vaporization of water are much worse than in linear cuts primarily because distortion occurs over a much smaller area than it does with a linear cut. Accordingly, once a portion along the perimeter/lateral inner surface of the through hole changes shape due to thermal interaction with the laser, the deformation cannot be recovered, nor accommodated anywhere due to lack of material,
ii) burnouts B appear at multiple locations along the perimeter of the inlet end of the through holes HD. Again, due to the very limited amount of material that is involved in the thermal interaction with the laser beam, upon vaporization of water or aqueous compounds from the tissue the latter is left with a rushing heat flow from the laser. As the laser is spot- or area-focused, the amount of liquid ware taking part of that heat flow the laser beam is markedly smaller as compared to that in a linear cut. This means that only a minor fraction of the total heat flow is spent for liquid ware evaporation, the remainder burdening on the bordering surfaces of the through hole. Consequence of this is - as said - the appearance of burnouts across the full hole array, both at the inlet end (figure 3) and at the outlet end (figure 5), and even more remarkably a deviation from the circular shape of the through hole which is particularly apparent form the outlet end views of figure 5. This is due to cutouts CO forming as the result of prolonged burning at certain burnout areas along the perimeter of the hole. The burning may get prolonged when the heat flow per unit volume is so intense that the implantable material simply cannot keep up, i.e. it cannot provide the required heat dissipation rates that would avoid thermal erosion of the material itself. The on-burning tissue cuts into the surrounding material, thus providing a droplet-like shape to the outlet end of the holes. As to the inlet end, the prolonged burning process may not necessarily result in cutouts: as the heat flow is higher than on the outlet end, the burning process erodes the tissue all around the inlet end, thus resulting in ring-shaped burnout areas at the inlet of each hole, wherein each area may itself be bordered by one or more burnouts B.

Both the burnout ring-shaped areas and the cutouts CO result in a marked deterioration of static resistance (such as, for example, resistance to tearing when sewing threads are tightened to connect portions of a prosthesis together), as well as fatigue resistance.

Conversely, in the embodiments of figures 4 and 6 all of the above effects are mitigated or eliminated. As to the inlet ends of the holes HW, there are no visible burnouts B around the perimeter of the holes, while at the outlet ends (figure 6) the contour of each hole is overall more regular as compared to figure 5, with no cutouts or distortions observed.

As already discussed with reference to figure 2, as glycerol is around 25% more dense than water, and at room temperature it is more viscous as well, when the "dry" implantable material ID is impinged upon by laser beams for providing the through holes HD, the share of the heat flow that is absorbed by glycerol is higher as compared to what happens with "wet" materials, and violent vaporization phenomena are substantially ruled out: glycerol provides a thermal sink that absorbs the local thermal shock generated by laser cutting process.

In this case, the effect of glycerol is even more apparent due to the limited size of the area involved. That is, the area of a through hole is so small as compared with that of a linear cut LCD, that the glycerol taking up heat flow from the laser is in an amount proportional to the very area. The thermal sink effect at the laser focusing spot or area is thus limited by the dimensions, but yet it avoids distortions at the hole end edge. Then, the glycerol filling the extracellular matrix at the surrounding areas to the hole(s) proves very effective in countering and avoiding prolonged burnouts and any cutout resulting therefrom.

The features of the processed element or component are thus not significantly different - if at all - from those of the starting material, and fatigue performances are not affected either. This is particularly important as through holes HD may be intended as sewing locations for routing of sewing threads: they must not tear up when stitching the components of the prosthesis together, nor do they have to break open under repeated loads from blood cycle operation.

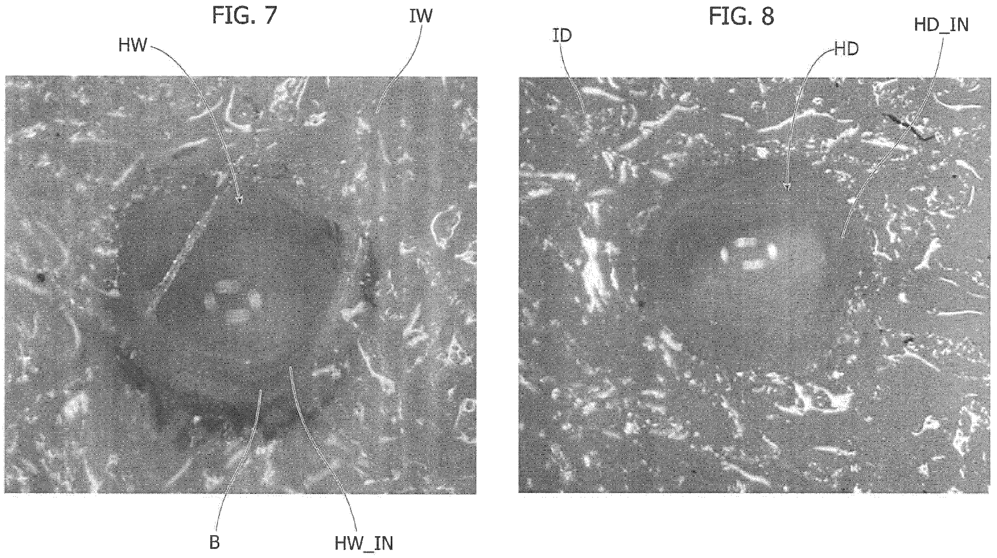

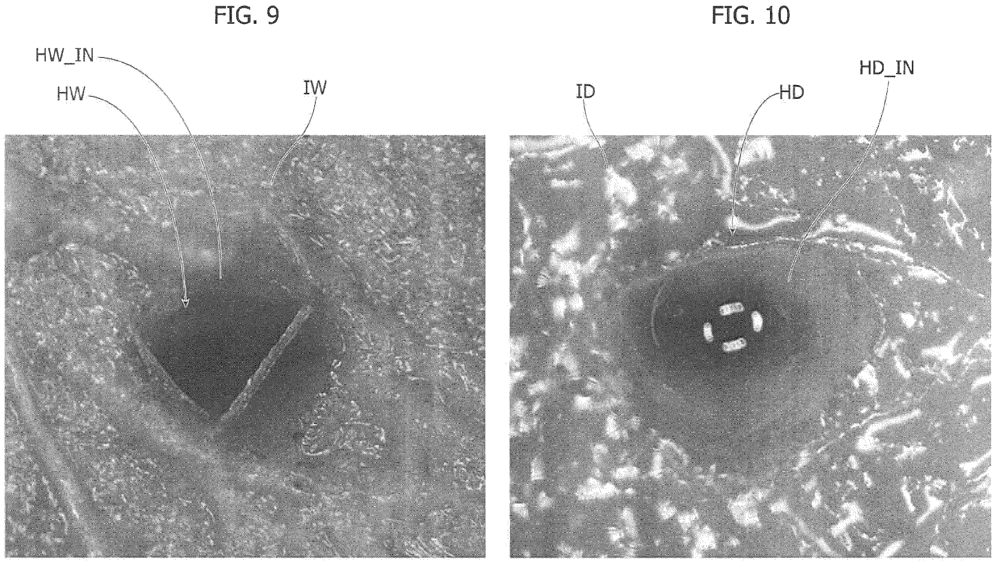

Confirmation of the performances of the method according to embodiments also come from the subsequent figures 8, 10, 12, 14 as compared to the counterpart "wet" material figures 7, 9, 11, 13. These figures refer to the same CO₂ laser treatment, but the operating parameters are set at intermediate values along the respective ranges. The views being magnified compared to those of figures 3 to 6 offer a greater level of detail that allows an even better appreciation of the effects of the method according to the embodiments. All the figures applying to comparative examples on "wet" implantable material show evidence of irregular contours, or even portions of tissues bridging together opposite surfaces of the through hole as a result of an incomplete burnout (figure 9). Thermal effects are only partly mitigated by the laser being operated at lower power.

No thermal effects are observed in the embodiments of figures 8, 10, 12, 14: the holes HD exhibit a largely more regular shape overall, with substantially no burnout areas.

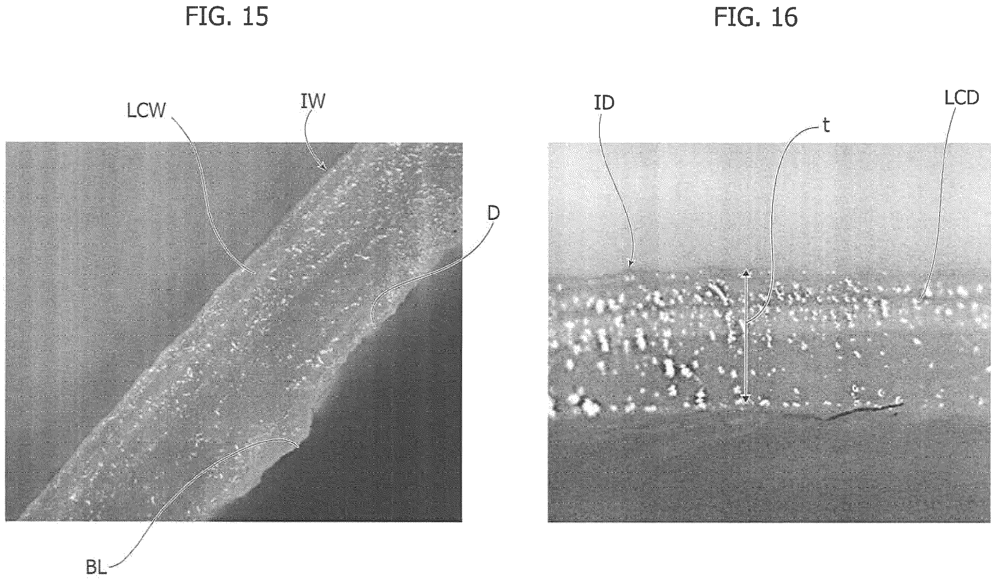

Figure 16 is representative of embodiments wherein the feature provided onto the implantable material is, similar to figure 2, a line feature, particularly a linear (straight line) cut. In these embodiments, however, the straight cut is provided by means of a femtosecond laser. Figure 15 is representative of the same operating conditions, but involves the use of "wet" bovine pericardium as implantable material, according to the prior art already referred to in respect of Figure 1.

The "wet" implantable material of figure 15 is, consistently with the foregoing description, identified by reference IW, while the "dry" implantable material of figure 16 is identified by the reference "ID". Accordingly, the linear cut is again identified by references LCW for the "wet" material and "LCD" for the "dry" material.

The femtosecond laser used with the embodiment of Figure 16, and the example of Figure 15, has a pulse duration of 400 femtoseconds (fs) or less, a wavelength of 520 mm, wherein wavelength may in general comprised between 400 nm and 600 nm, and a lens focal length of 100 mm, wherein lens focal length may in general be comprised between 90 mm and 110 mm.

Operating parameters applying to the embodiment of figure 16 correspond to the following
i) Power: 4W to 6W, preferably 6W
ii) Velocity: 1000 mm/s to 2000 mm/s, preferably 1500 mm/s
iii) Frequency: 80 kHz to 100 kHz, preferably 100 kHz
iv) Number of passes: 60 to 100, preferably 80
v) Lens focal length: 100 mm.

A femtosecond laser interacts with the implantable material in a way closer to mechanical ablation than thermal ablation. A femtosecond laser may be used, in embodiments, to provide non-through features of the types listed in the foregoing description. This substantially eliminates thermal defects such as burnouts or cutouts, and the distortions from quickly evaporating water, but still geometry problems remain when "wet" materials are used.

As visible in figure 16 when compared to figure 15, the surface exposed following cutting exhibits an essentially pristine structure, as if it was cut by a mechanical tool. All of the pericardium layers are perfectly visible across the thickness t, and overall there is no alteration whatsoever to the cutout component as compared to the starting material.

As to the comparative example of figure 15, the cut profile shows evidence of geometric singularities such as bulges BL and dents D, and the layers are less visible - if at all - than figure 16.

The advantage may not reside in thermal sink properties of glycerol, but rather on the structural coherence the same provides to the "dry" implantable material ID. To provide a comparison, when interacting with a femtosecond laser, the "wet" material IW has an extracellular matrix that may be regarded as a dry sponge. Water or aqueous compounds in general do not fill the extracellular matrix as water would do with an actual soaked sponge, nor would water or aqueous compounds provide any additional structural contribution to the extracellular matrix due to the comparably low density and viscosity thereof.

With a glycerol treated implantable material ID, glycerol - having its higher density and viscosity - fills the interstitial space in the extracellular matrix and renders the extracellular matrix a very cohesive and solid structure, much different from the fundamentally alveolar and relatively "fragile" structure of the "wet" material.

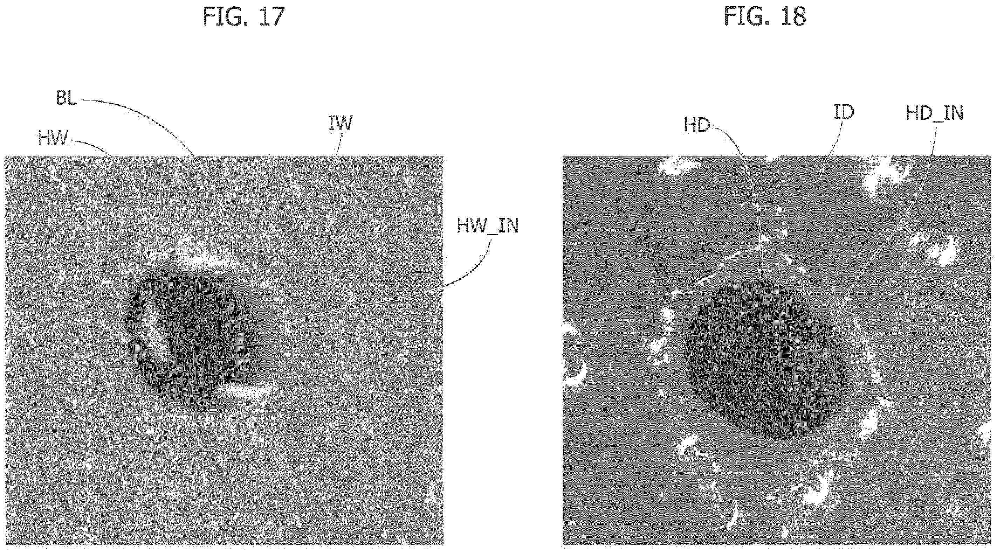

Figure 18 is representative of embodiments wherein the feature provided onto the implantable material is a closed loop feature, particularly the cutting of a through hole to provide a sewing reference. The through hole is cut by means of the femtosecond laser.

Figure 17 is representative of the same operating conditions, but - similar to figures 3, 5, 7, 9, 11, 13, 15 - they involve the use of "wet" bovine pericardium as implantable material, according to the prior art. The "wet" implantable material of figure 17 is again identified by reference IW, while the "dry" implantable material of figures 18, is again identified by the reference "ID".

Accordingly, the through holes are identified by references HW for the "wet" material and "HD" for the "dry" material. Whether made on a wet or dry material, the holes appearing in the figures are also identified based on the visible rim being the inlet end or the outlet end of the laser beam, which in this case is the sole inlet end. Consistently with the above, inlet ends are identified by reference HW_IN for the "wet" material, and by reference HD_IN for the "dry" material.

The femtosecond laser used with the embodiment of Figure 18, and the example of Figure 17 is the same as that used with the linear cuts LCW and LCD of figures 15 and 16, i.e. has a pulse duration of 400 femtoseconds (fs) or less, a wavelength of 520 mm, wherein wavelength may in general comprised between 400 nm and 600 nm, and a lens focal length of 100 mm, wherein lens focal length may in general be comprised between 90 mm and 110 mm.

Operating parameters applying to the embodiment of figure 18 (as well as to the example of figure 17) are however different from those used on the implantable materials IW and ID of figures 15, 16, and are chosen with a view of the specific feature being provided, i.e. a through hole.

This being said, the parameters correspond to the following
vi) Power: 4W to 6W, preferably 6W
vii) Velocity: 1000 mm/s to 2000 mm/s, preferably 1500 mm/s
viii) Frequency: 80 kHz to 100 kHz, preferably 100 kHz
ix) Number of passes: 80 to 180, preferably 150.
x) Lens focal length: 100 mm.

With reference to figures 17, 18, although the defects on the through hole HW are less extensive than with CO₂ laser cut, the outline at the inlet end still exhibits a bulge BL. No such defects are visible on the through hole HD of figure 18, essentially for the very reasons as discussed in respect of the linear cut. At least roughly, the difference in the interaction with femtosecond laser between "wet" materials and "dry" materials is the same difference with cutting a crispy bread loaf ("wet") and a butter slab ("dry"). While they both get cut acceptably, the butter allows for a smoother cut on account of the much more cohesive structure thereof.

The application of the method according to the embodiments to the manufacture of cardiovascular prostheses may allow, almost single handedly, to overcome all of the drawbacks and technical difficulties encountered with the prior art. Components for heart valve prostheses may be cut out of "dry" implantable material patches, whatever the contour thereof, by means of laser cutting, and may at the same time be provided with stitching references in the form of through holes, all of this with almost untouched mechanical properties compared to the starting material.

Not only this, when comparing components for heart valve prostheses and heart valve prostheses made according to the method of the embodiments with components and prostheses made in accordance with the prior art the difference is staggering. Especially as far as stitching is concerned, stitching patterns get overall more regular and repeatable thanks to the method of the embodiments, and the workers assembling the prosthesis are no longer burdened by the task of piercing the implantable material themselves with a stitching needle. Piercing by a needle inherently creates irregularly shaped holes and slits that in any case have worse fatigue performances than a through hole which is laser cut as a feature into an implantable material.

In summary, prostheses manufactured according to the method of the embodiments have better mechanical strength (both static and fatigue), can be dry stored for long periods of time, and when implanted take benefit from a much extended service life on account of better mechanical properties, all to a higher safety for the patient.

The disclosure that follows includes embodiments of cardiovascular prostheses featuring one or more elements or components made in accordance with the method disclosed herein. An exemplary such prosthesis is disclosed in European Patent no. EP 1 690 515 B1 in the name of the same Applicant, which is incorporated by reference herein, and another such prosthesis is disclosed in PCT application no. PCT/IB2018/053640 in the name of the same Applicant, also incorporated by reference herein and summarized in the brief description below.

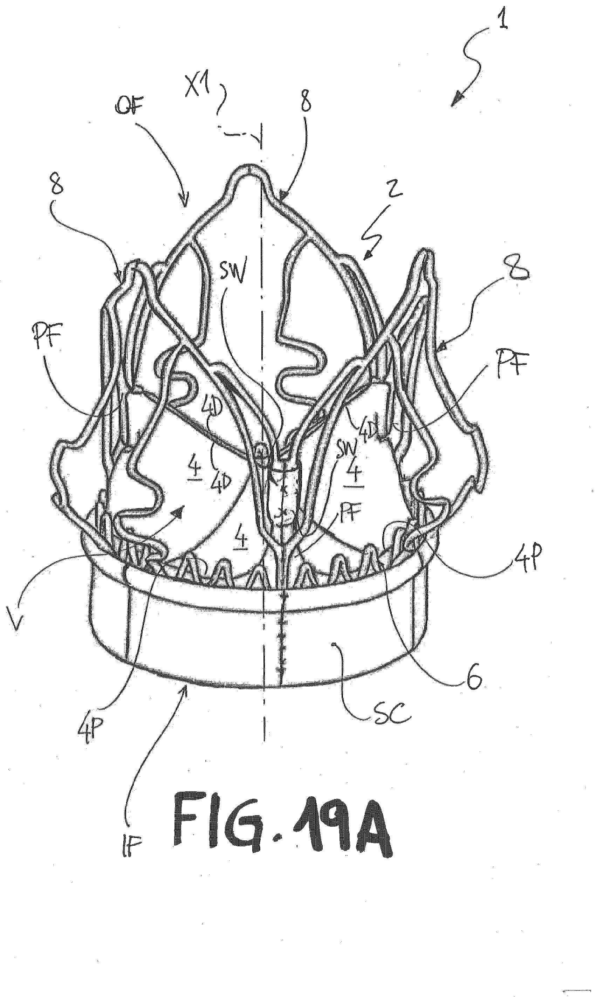

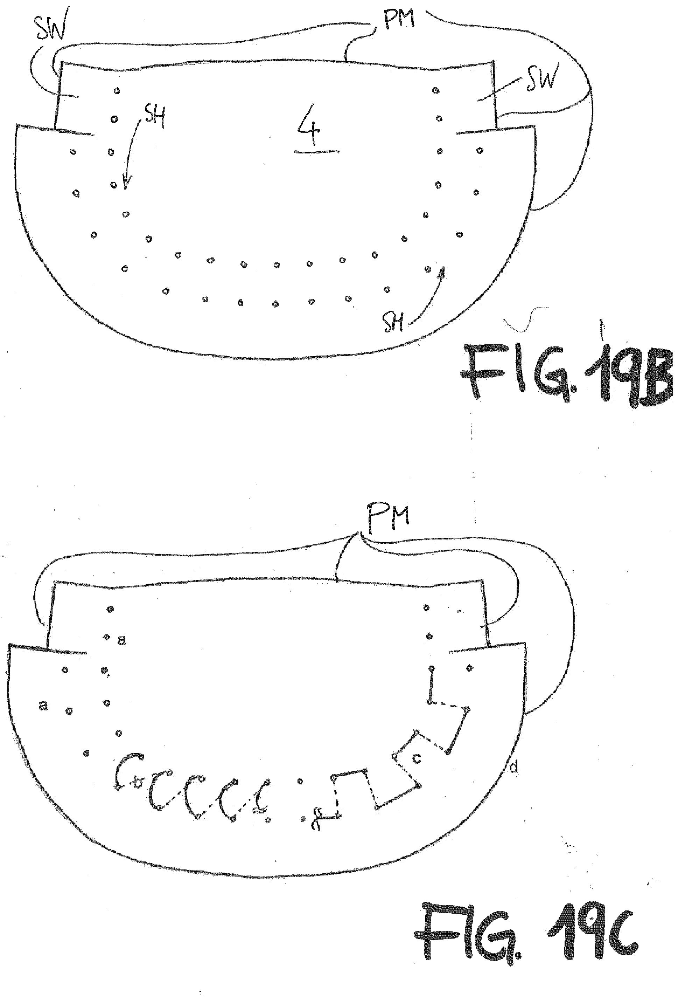

With reference to Figure 19A, reference number 1 designates as a whole a cardiac valve prosthesis according to various embodiments of the disclosure. The description that follows discloses i.a. a method of making the cardiac valve prosthesis (or other implantable vascular prostheses) by taking advantage of the method of providing features on an implantable material according to embodiments. The implantable vascular prostheses, particularly the cardiac valve prostheses of the embodiments may also be provided within kit including a delivery instrument for the prosthesis and a dry-storage container. Owing to the features of the material ID, storage in a preservation solution is not required. A storage container for embodiments of the kit is disclosed in PCT application no. PCT/IB2018/053645 in the name of the same Applicant, incorporated by reference herein. A delivery system for embodiments of the kit is disclosed in PCT application no. PCT/IB2018/053646 in the name of the same Applicant, also incorporated by reference herein. The kit may also include a loading facility such as that disclosed in PCT application no. PCT/IB2018/053648 in the name of the same Applicant, also incorporated by reference herein. In embodiments of the kit. The loading facility may be used to facilitate loading of the prosthesis 1 onto a delivery instrument.

The cardiac valve prosthesis 1 includes an armature 2 for anchorage of the valve prosthesis at an implantation site. The armature 2 defines a lumen for the passage of the blood flow and has a longitudinal axis X1.

The prosthesis 1 also includes a set of prosthetic valve leaflets 4 supported by the armature 2 and configured to move, under the action of blood flow (which has a main flow direction roughly corresponding to that of the axis X1): in a radially divaricated condition to enable the flow of blood through the lumen in a first direction, and in a radially contracted condition, in which the valve leaflets 4 co-operate with one another and block the flow of blood through the prosthesis 1 in the direction opposite the first direction. This is commonly referred to as leaflet coaptation.

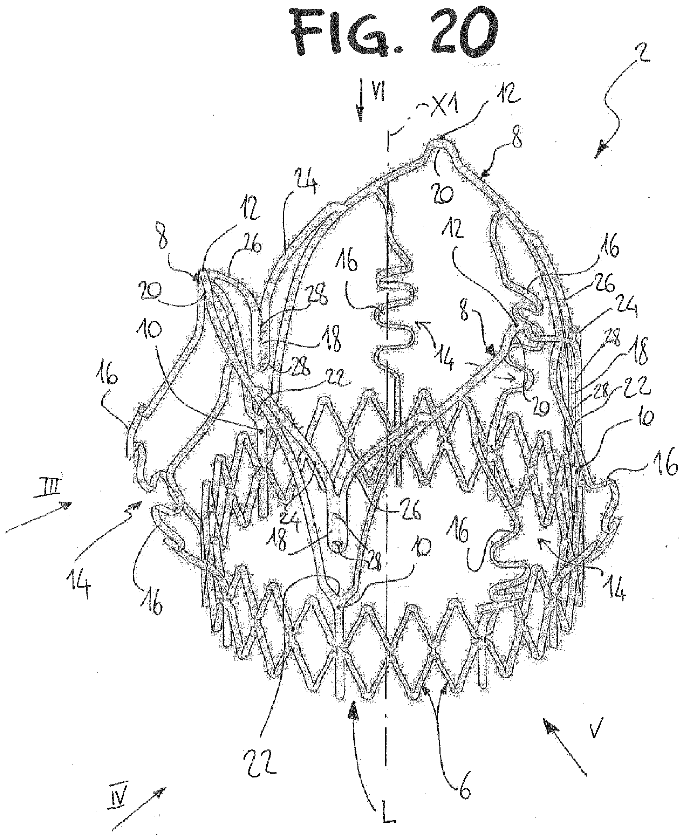

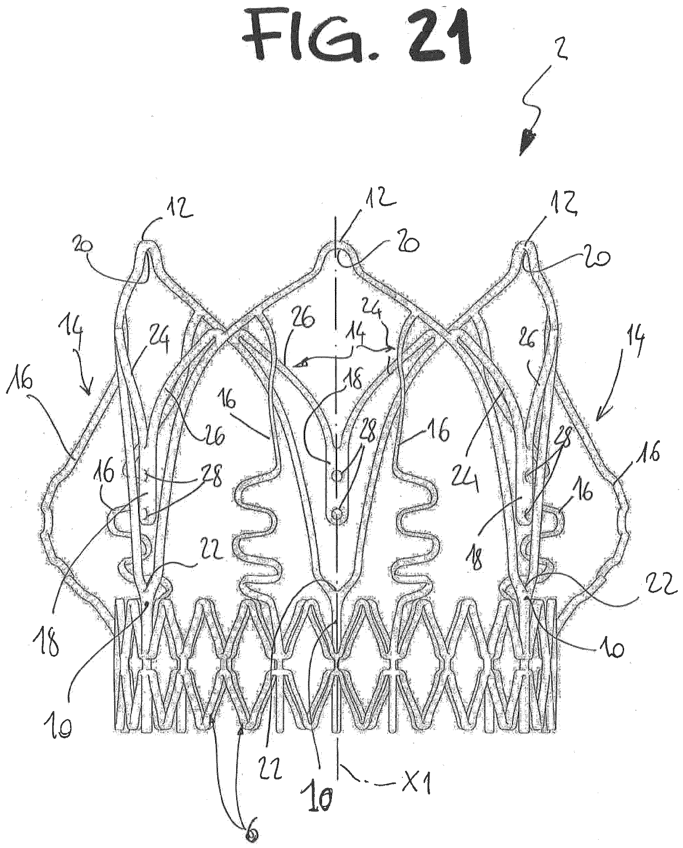

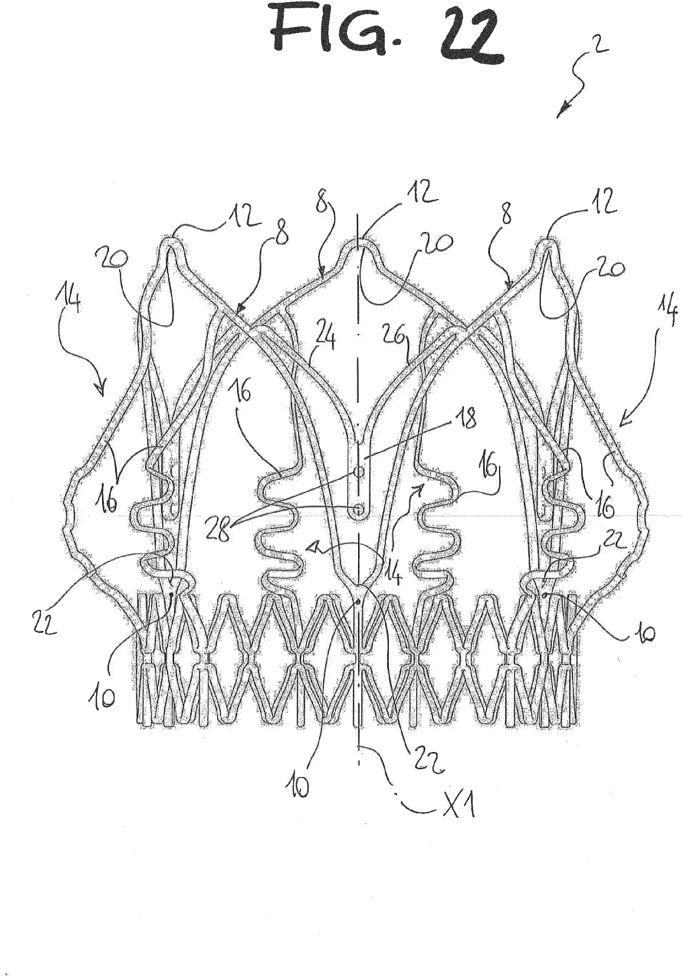

With reference to Figures 20-24, in embodiments the armature 2 includes an annular part 6, and a pattern of arched struts 8 carried by the annular part 6. The annular part has a structure which can expand from a radially contracted condition, associated to delivery of the prosthesis to implantation site, to a radially expanded condition wherein the prosthesis is withheld at the implantation site. In these embodiments, the annular part may have a mesh structure including an annular pattern of multiple strut clusters (cells) having polygonal shape (hexagonal, rhomboidal, etc.).

In embodiments, the features of the valve leaflets 4 are provided by the method according to embodiments of this disclosure. As regards the construction of the set of leaflets 4 (also referred to as or valve sleeve), in embodiments the prosthetic valve is made with three separate leaflets. Each leaflet is obtained from one sheet of pericardium trimmed accordingly to Figure 19B, i.e., according to a substantially lobe-shaped figure. Each trimmed leaflet features two patterns of sewing holes SH and two side wings SW.

In embodiments, each leaflet is provided by the method disclosed herein starting from a sheet of dry pericardium according to the embodiments disclosed in the foregoing. The perimeter PM of each leaflet is exemplary of a line (and through) feature according to the method herein cut through the dry pericardium by means of a CO₂ laser or a femtosecond laser, thereby obtaining a cut perimetral edge having the features shown in figures 2 (CO₂ laser) and 16 (femtosecond laser). Cutting parameters according to the embodiments disclosed herein may be used. Through laser cut may also extend into the perimeter edge as much as needed in order to provide the side wings SW.

The patterns of sewing holes SH are provided as an example of a closed loop (and through) feature according to the method herein, and are cut according to the same method with a final shape exemplified by figures 4, 6, 8, 10, 12, 14 (CO₂ laser) or 18 (femtosecond laser).

The sewing holes SH are then sewn together forming a sewn stiffer fold which follows the leaflet profile at the root thereof, thereby forming the cusp.

The two side wings SW enable connection of the valve to supporting posts in the valve armature. The sewn fold has the purpose to bias the cusp inwardly thereby encouraging leaflet coaptation, and to avoid contact between the armature 2 and the valve leaflets 4 avoiding the risk of abrasion due to repeated impact against the armature 2, which, in some embodiments, is a metal material.

The two patterns of holes SH may be sewn together using a suture thread coated with a film of biocompatible material or PET thread or PTFE filament.

This may include in embodiments using the sewing holes SH to sew together the leaflet 4 into a valvular sleeve prior to sewing the valvular sleeve to the armature 2.

The sewing pattern may be varied to accommodate the directional differences in the forces exerted at each point of the stitches, prevent the stitches from triggering fatigue fracture lines.

Preferably the stitching follows the pattern identified by letter "C" in Figure 1C (alternate inner and outer surface stitches). The three leaflets 4 are then joined at the side wings SW and stitched together along the leaflet post line, i.e., at the interface of adjacent side wings SW, thereby forming a conical duct.

The side wings SW, which allow a slack of material that protrudes outwardly of the duct, are then fixed to the armature posts, which are fully wrapped by the side wings SW.

The extra tissue skirt below the stitching holes SH allows leaflet fixation to the inflow ring 6 of the stent, by mean of a stitching line. Additionally, in some embodiments one - preferably the lower one or both of the patterns SH may be stitched to the armature at the annular part 6.

A strip of pericardium is finally stitched to the inflow ring 6 outwardly of the same, defining the sealing cuff SC. The strip can be folded on its outflow end to provide a sealing collar along the valve perimeter, or a second strip can be connected by means of a circumferential stitching line to the first strip, to realize a sealing circumferential collar.

Similarly to the leaflets 4, the strip of pericardium defining the inflow ring 6 may itself be cut out of a dry sheet of pericardium according to embodiments herein. The perimeter of the strip is another example of a line (and through) feature that may be cut by the method.

As mentioned, the method disclosed herein also allows to provide non-through features on dry pericardium sheets, for example surface sculpturing features or ablation of a part of a layer overall of biological material from the dry pericardium sheet ID, for example to provide differentiated local thicknesses to the pericardium sheet ID to address specific needs.

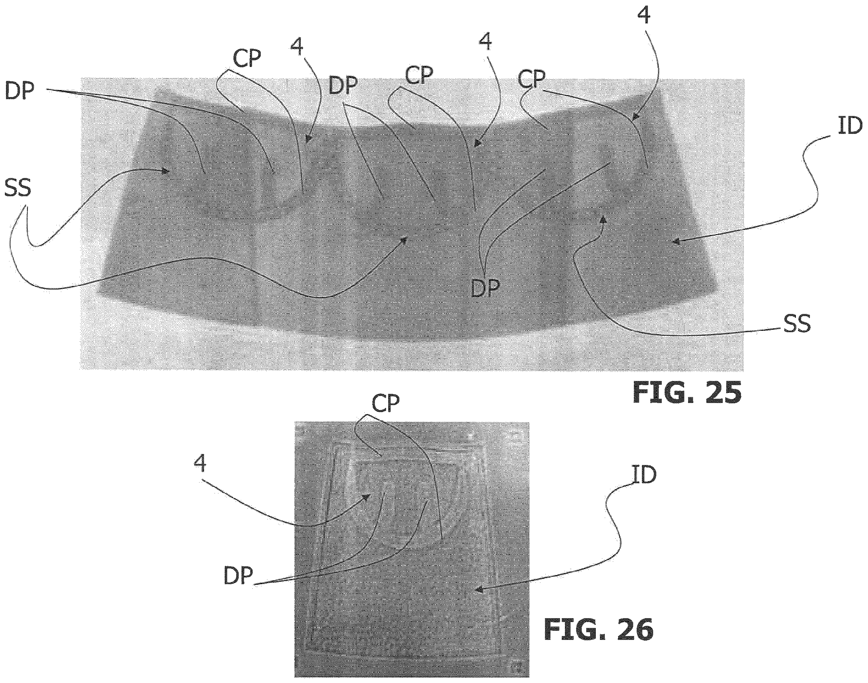

An exemplary embodiment of a surface sculpturing provided by the method according to embodiments herein is shown in figures 25 and 26. In such embodiments, the surface sculpturing may include one or more sculpturing patterns provided as a sequence of modular sculpturing units. In embodiments, a first sculpturing pattern is provided as an arched contour pattern CP extending along the perimeter of the leaflets 4 and intended to increase fatigue resistance of the leaflets 4 during coaptation. In embodiments, the contour pattern CP is provided as a result of selective ablation of layer(s) of the leaflet within a perimeter line defined by the contour pattern CP, so that the latter is overall thicker than the leaflet area enclosed thereby. Due to the shape of the contour pattern, increased thickness is crucially located at a fluidodinamically distal edge (outflow end) of the leaflets and at a fluidodinamically proximal edge (inflow end) of the leaflets, viz. the arched "root" of the leaflets, which happen to be the portions most subject to fatigue stress. The terms "fluidodynamically proximal" and "fluidodynamically distal" as used herein refer to the free flow direction of the blood through the prosthesis, a direction that is bottom up as viewed in the figures of the annexed plate of drawings.

In embodiments, a second sculpturing pattern includes pairs of deformation patterns DP. The deformation patterns DP help create preferential folding directions on each leaflet 4 to prevent decay in leaflet performances over time, viz. to prevent "freezing" of the shape of the leaflet at locations away from a fluidodinamically distal edge, i.e. at locations near a fluidodinamically proximal edge. Again, the deformation pattern results, in embodiments, from ablation of surrounding portions of dry pericardium sheet ID so that the deformation patterns DP overall edge out of the surrounding areas.

The deformation patterns DP encourage folding deformation of the valve leaflets 4 during coaptation, that is, the surrounding portions of the leaflet will be encouraged to fold about - as much as allowed by the overall arrangement of the three leaflets - the thicker deformation patterns DP to maintain a maximum of the leaflet area in active conditions throughout the service life of the prosthesis.

Depending on the needs, each sculpturing pattern CP, DP (including ablation patterns) may be provided either edging out of surrounding areas as disclosed above, or else embossed relative thereto (as a channel-like feature). In this regard, figure 25 is representative of embossed sculpturing patterns CP, DP, while figure 26 is representative of edging out sculpturing patterns CP, DP.

In embodiments, the embossed sculpturing patterns CP, DP of figure 25 are provided as a sequence of circles or closed shape figures chained or cascaded together to provide a continuous overall outline. In the embodiment in the figures, the sculpturing includes sequences of chained or cascaded circles engraved in the pericardium sheet ID by the method according to the embodiments herein. Figure 25 is representative of sculptured (non-through) features provided as a sequence of circles chained together and sculptured into the pericardium layer ID by femtosecond laser treatment, while the femtosecond laser treatment provides the ablation of the pericardium in areas surrounding the patterns CP, DP in figure 26.

The femtosecond laser used with the embodiments of figures 25 and 26 may be the same as disclosed in the foregoing, i.e. it has a pulse duration of 400 femtoseconds (fs) or less, a wavelength of 520 mm, wherein wavelength may in general comprised between 400 nm and 600 nm, and a lens focal length of 100 mm, wherein lens focal length may in general be comprised between 90 mm and 110 mm.

Operating parameters applying to the embodiments of figures 25, 26 correspond to the following
i) Power: 2W to 6W
ii) Velocity: 7000 mm/s to 9000 mm/s
iii) Frequency: 200 kHz to 500 kHz
iv) Number of passes: 10 to 50
v) Lens focal length: 100 mm.

The provision of sculpturing patterns CP, DP as embossed features may be desirable, for example, whenever certain elements (say, the leaflets 4) of a cardiovascular prosthesis are to be made out of a relatively thick or thicker than standard pericardium. In that case, fatigue resistance may not be an issue considering that the leaflet would be, so to say, beefed up as compared to a standard manufacturing, but leaflet functionality may be hampered by the extra thickness. For this reason, and with reference to figure 25, the patterns CP, DP may be provided either at the exact locations as the edging out counterparts in figure 26 or at different locations with the aim of increasing deformability and/or flexural performances of the leaflet. In the embodiment of figure 25, the provision of embossed patterns as the leaflet contour - pattern CP - and as deformation patterns - pattern DP - within the leaflet contour may ensure that the leaflet does not exhibit excessive stiffness at the relevant locations, thereby compromising the very leaflet coaptation properties.

Another reason to provide embodiments with embossed patterns (e.g. deformation pasterns DP) is that of sculpturing preferential folding or collapse lines to facilitate crimping, whether or not the pericardium is of standard thickness or high thickness.

More in general, surface sculpturing features provided by the method according to embodiments herein may be used to vary the local thickness of the leaflet and/or the local pattern of the leaflet surface to provide the leaflet with desired properties at a specific location. The surface sculpturing patterns CP and DP are one possible example of this.

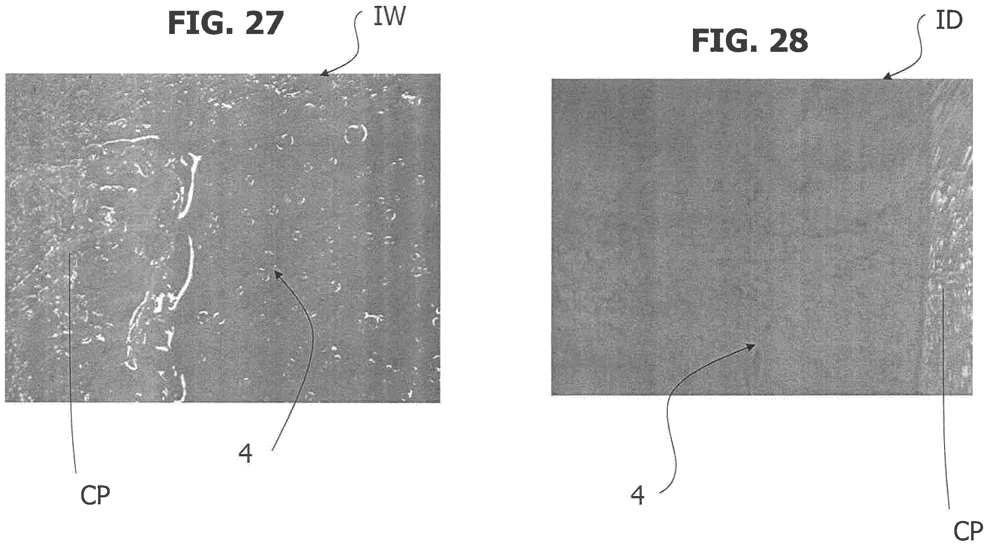

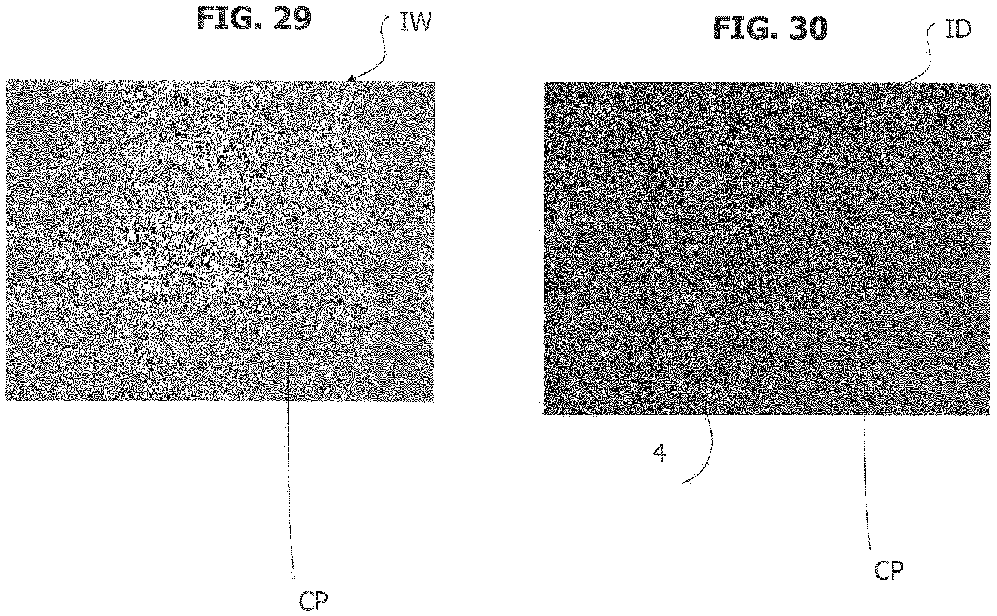

Figures 28 and 30 are representative of a CO₂ laser sculpturing (ablation) on a section of a valve leaflet 4 made of dry pericardium ID, again to provide a contour pattern CP. Figure 30 shows the rehydrated dry pericardium sheet. Compared to the same pattern provided by means of CO₂ laser on a wet pericardium sheet IW in figures 27 and 29, the sculptured dry pericardium sheet exhibits unprecedented neat sculptured contours.

The CO₂ laser used with the embodiment of Figures 28, 30, and the example of Figures 27, 29, is the same as that used with the linear cuts LCW and LCD of figures 1 and 2, i.e. it has a wavelength of 10600 nm, wherein the wavelength may in general be comprised between 9000 nm and 12000 nm, a maximum rated power of 30 W, wherein maximum rated power may in general be comprised between 15 and 40 W, a maximum rated processing velocity of 1800 mm/s, wherein the maximum rated processing velocity may in general be comprised between 1500 mm/s and 2300 mm/s, and a lens focal length of 25,4 mm, wherein the lens focal length may in general be comprised between 10 mm and 50 mm.

Operating parameters applying to the embodiment of figures 28, 30 (as well as to the example of figures 27, 29) are however different due to the specific features and requirements of a surface sculpturing (non-through feature).

This being said, the parameters correspond to the following
i) Power: 20% to 70% of maximum rated power
ii) Velocity: 20% to 65% of maximum rated processing velocity
iii) Frequency: 300 ppi to 1000 ppi (points per inch)
iv) Number of passes: 1 to 10
v) lens focal length: 25.4 mm.

The prosthetic leaflets 4 may be in any number compatible with operation as replacement heart valve. In some embodiments, the set includes a pair of leaflets. In some embodiments, such as that shown in the figures, the set includes three prosthetic valve leaflets 4 (e.g. for an aortic valve prosthesis). In some embodiments, the set may include four leaflets 4.

Each valve leaflet 4 includes a fluidodynamically proximal edge 4P with an arched pattern, which extends from a base portion at the upper pattern SH and along two adjacent pleat formations PF, and a fluidodynamically distal edge 4D which extends towards the central orifice of the prosthesis 1 so as to be able to co-operate with the homologous edges of the other valve leaflets 4.

During operation (heart cycle) the valve leaflets 4 experience deformation, divaricating and moving up towards the armature 2 so as to enable free flow of the blood through the prosthesis.

When the pressure gradient, and hence the direction of flow, of the blood through the prosthesis tends to be reversed, the valve leaflets 4 then move into the position represented in Figure 1A, in which they prevent the back flow of the blood through the prosthesis.

The pattern of arched struts 8 includes proximal ends 10 connected to the annular part 6, and distal ends 12 spaced axially from the proximal ends 10 and arranged at an end of the armature 2 opposite the annular part 6. In embodiments, the distal ends 12 coincide with distal ends of the armature 2, and in embodiments where the distal end of the armature 2 coincides with a distal end of the prosthesis 1 as a whole, the distal ends 12 coincide with a distal end of the prosthesis as well (this is the case of at least some of the embodiments depicted in the figures).

Owing to this layout, in embodiments, the prosthesis 1 includes an inflow portion IF essentially corresponding to the annular part 6 (whether or not covered by the sealing cuff SC), and an outflow portion OF corresponding essentially to the distal region of the armature, i.e. that where the distal ends 12 are arranged.

The armature 2 further includes a plurality of sets 14 of anchoring formations 16 configured to protrude radially outwardly of the annular part 6, each set 14 being supported by at least one of the annular part 6 and a corresponding arched strut 8, and a plurality of support posts 18, each supported by adjacent arched struts 8, wherein the sets 14 of anchoring formations 16 alternate with the support posts 18 around the longitudinal axis X1. In embodiments the support posts 18 are advantageously cantilevered to adjacent arched struts 8 and are configured as fixing locations for the prosthetic valve, specifically for the pleat formations PF at the commissural points of the valve.

In this regard, the posts 18 are wrapped by adjacent side wings SW previously stitched together during assembly of the prosthetic valve. The leaflets 4 may in embodiments be sewn together and to the support posts 18 in a single motion, while in other embodiments the leaflets 4 are first sewn together to form the valvular sleeve, then the valvular sleeve is sewn to the supporting posts.

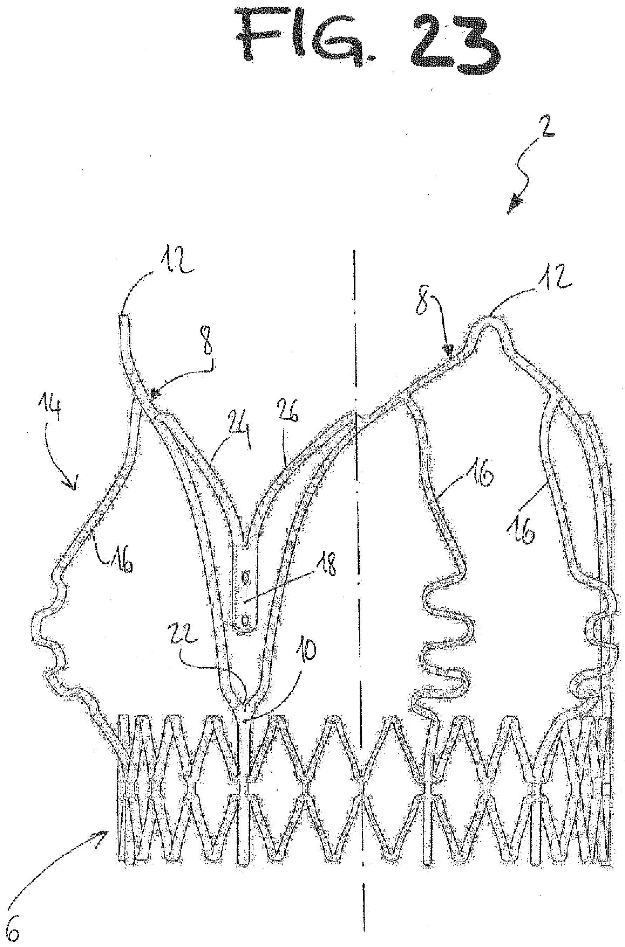

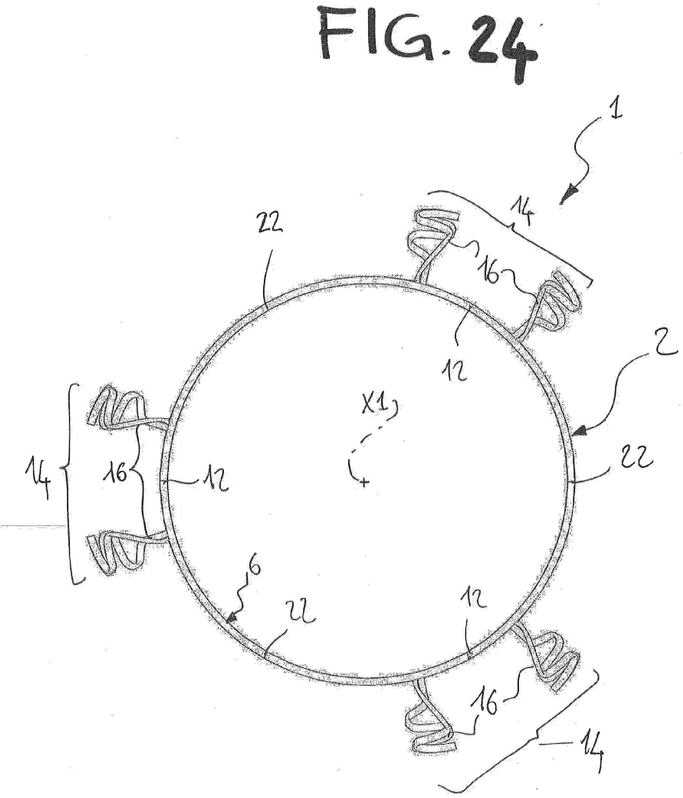

Referring again to Figures 20-24, in embodiments each arched strut 8 extends from a first proximal end 10, to a distal end 12, then to a second proximal end 10 in a valley-peak-valley sequence, wherein valleys are located at the proximal ends 10, and peaks are located at the distal ends 12. In embodiments the pattern of arched struts includes three adjacent and preferably identical arched struts 8 (such as in the figures). With reference to Figure 23 and Figure 24, in embodiments, the arched struts 8 extend within the boundaries of the inner and outer surfaces of the armature 2, so as to be substantially free of any protrusion relative to those surfaces. In other embodiments the arched struts may have an offset from the inner and outer surfaces of the armature 2, meaning by this they may either protrude radially inwardly or radially outwardly of two cylinder surfaces tangent to the inner and outer surfaces of the armature 2 (in the view of Figure 24, this would correspond to a radially inward or a radially outward offset from the circular perimeter of the lumen defined by the armature 2.

In embodiments, the arched struts are sized and dimensioned so as to have a variable curvature between a proximal end 10 and a distal end 12, for example with the arched shape starting with a 45 degrees tangent at the proximal end 10, and ending up with an 80 degrees tangent at the distal end, the angle being measured relative to a direction parallel to the axis X1

In some embodiments, the arched struts 8 are sized and dimensioned so as to exhibit appreciable variations in curvature between proximal and distal ends 10, 12. The pattern of arched struts 8 includes distal portions 20 located at the distal ends 12, and inter-strut portions 22 located at the proximal ends 10. The distal portions 20 may be shaped so as to provide a marked local variation in the shape of the strut, for example by exhibiting a C-shape as shown in the figures. The distal portions 20 may provide coupling locations for other devices such as a valve holder or a hub of a carrier portion of a delivery catheter. In other embodiments, the distal portions 20 may be provided as closed-loop structures such as eyes or eyelets. Note also that closed loop structures may be provided at the annular part 6 (either as part of the armature 2 or on the sealing cuff SC, for example as loops made of yarn and weaved through the cuff SC) as coupling elements intended to be engaged e.g. by valve loading or crimping facilities or instruments.

In embodiments, the inter-strut portions 22 are essentially V-shaped and are defined by the roots of the adjacent arched struts departing from the same proximal end 10. In some embodiments, the inter strut portions 22 may exhibit a Y-shape or a U-shape. An example of a Y-shape is shown in the figures (particularly Figures 20-23) wherein each inter-strut portion 22 extends through the mesh of the annular part 6. In these embodiments, the mesh of the annular part 6 is provided as a sequence of rhomboidal strut clusters (cells) sequentially connected to each other at endpoints of a diagonal line (such as the shortest diagonal) and exhibiting accordingly an identical circular pattern of free ends on opposite sides of a circumference extending through the sequence of the connection points. The Y-shaped inter-strut portion 22 is thus integrally formed at a selected connection point between two adjacent rhomboidal strut clusters, and may extend no further than the proximal end of the armature 2.

In embodiments the strut clusters may be arranged according to an arrow shape, i.e. defined by two axially staggered sinusoidal patterns, circumferentially in phase, bridged by longitudinal struts.

In embodiments, the support posts 18 are angularly arranged at an inter-strut location, i.e., a circumferential location arranged at an area where an inter-strut portion 22 (as well as - accordingly - a proximal end 10 shared by two adjacent arched struts 8) is provided. The support posts may be provided as cantilevered to both the adjacent arched struts 8 intervening at an inter-strut portion 22 via a first and a second cantilever struts 24, 26, each connected to a corresponding one of said adjacent arched struts 8 as shown in the figures. In some embodiments, each cantilever strut 24, 26 may be a twin strut.

The cantilever struts 24, 26 merge into each corresponding post 18 starting from locations on respective arched strut 8 approximately halfway through the portion of the arched strut 8 extending from a proximal end 10 to a distal end 12. Note that in other embodiments the support posts 18 may be cantilevered to the annular part 6, for example by being formed integrally with the inter-strut portion 22 (which in this case will exhibit a trident shape).

The connection points at which the Y-shaped inter-strut portion 22 is formed may be chosen so that the same portions are evenly spaced (angular-wise) around the axis X1. The same applies to the support posts 18, which may be arranged so as to be evenly spaced (angular-wise) around the axis X1.

In some embodiments shown in the figures, the armature 2 comprises three arched struts 8, three posts 18 spaced 120° around the axis X1, and three sets 14, so that the sequence around the axis X1 is post 18 - set 14 - post 18 - set 14 - post 18 - set 14 (in this sense, even the struts 8 and the sets 14 do follow a 120degree-like distribution). In embodiments the three sets 14 include each a pair of anchoring formations 16, wherein each set 14 (and accordingly each anchoring formation 16) extends bridge-wise between the annular part 6 and the corresponding arched strut 8. In embodiments, each pair of anchoring formations 16 extend bridge wise at an intra-strut location, that is a location within an arched strut 8 and as such comprised between two proximal ends 10 of the same arched strut 8 and under a distal end 12/distal portion 20. In other words, each set 14 of anchoring formations 16 extends bridge-wise from the arched strut 8 to a portion of the annular part 6 comprised between two proximal ends 10.

The support posts 18, accordingly, are arranged at an inter-strut location (e.g. above the inter-strut portion 22 as shown in the figures) so that the sequence post 18 - set 14 - post 18 - set 14 - post 18 - set 14 is provided, location-wise, as inter-strut-intra-strut - inter-strut - intra-strut - inter-strut-intra-strut.

In embodiments, the support posts 18 are provided with bores 28 configured for receiving sutures or stitches that fix the commissural portions (pleat formations of the valvular sleeve) to the posts 18, hence fixing the prosthetic valve to the armature 2. In some embodiments, the prosthetic valve is fixed to the support posts 18 with the same being completely outside of the valve. Sutures or stitches are routed through the bores 28 and through the valve layers contacting the corresponding post 18 from inside of the lumen. In other embodiments, the posts 18 may be wrapped by the valve layers - particularly by the commissural points of the valve - so that the valve is at least partially "outside" of the posts 18.

In embodiments, the anchoring formations may include a serpentine or otherwise weaving portion between opposite ends thereof. Such a serpentine or otherwise weaving portion is intended to provide a larger footprint to the anchoring formation at the interface with a Valsalva Sinus, and additionally it enables the bulging of the anchoring formations to a diameter larger than the inflow diameter, typically 1.4 or 1.5 times the inflow diameter.

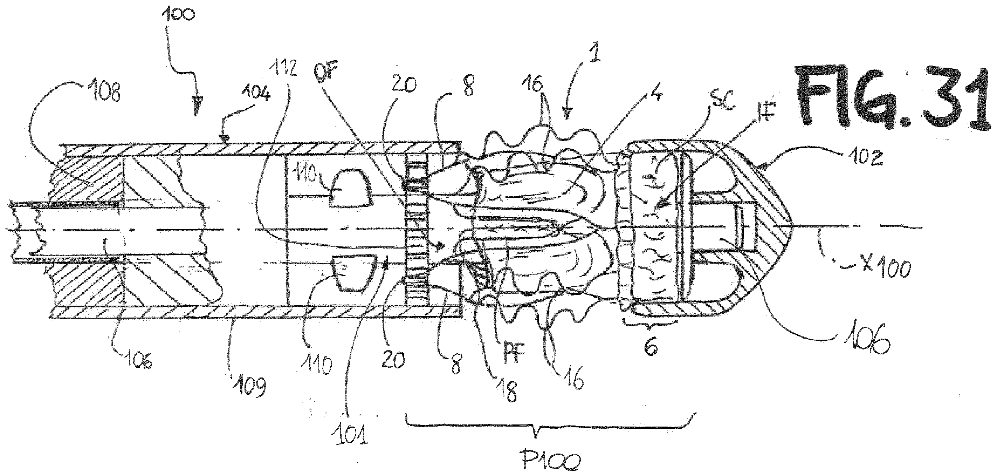

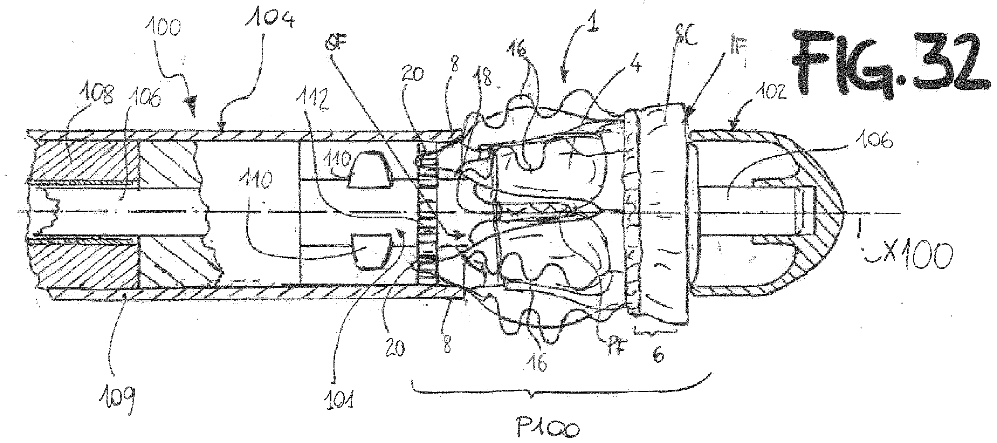

According to embodiments, a prosthesis 1 including portions thereof made of "dry" pericardium (or more in general of an implantable material ID comprising a decellularized sterilized mammalian tissue having an extracellular matrix, wherein a plurality of interstitial spaces of the extracellular matrix include a solution of one or more polyols) that has been subjected to a laser processing by the method according to embodiments therein provides unprecedented advantages in terms of storage. Since the "dry" pericardium/implantable material as disclosed herein does not require preservation in a liquid sterile solution, and - instead - it only requires an ethylene oxide sterilization similar to that commonly practiced on surgical instruments, various embodiments herein - with specific reference to figures 31 and 32 - may comprise a kit including a delivery instrument 100 with a heart valve prosthesis 1 pre-mounted/pre-loaded thereon, wherein the prosthesis 1 includes the leaflet 4 made of "dry" bovine pericardium or more in general of the implantable material ID comprising a decellularized sterilized mammalian tissue having an extracellular matrix, wherein a plurality of interstitial spaces of the extracellular matrix include a solution of one or more polyols.

With reference to figures 31 and 32, a delivery instrument 100 according to embodiments of the kit includes a shaft having a longitudinal axis X100, a handle at a first end of the shaft; and a carrier portion P100 at a second end of the shaft. Figures 31 and 32 only show the carrier portion P100 in that the handle-shaft-carrier portion layout is per se known in the art.

The carrier portion P100 is configured for holding an expandable heart valve prosthesis, such as the prosthesis 1 at least partly in a radially collapsed condition for delivery to the implantation site.

The carrier portion P100 may include a hub 101 fixed to the handle via the shaft, a first deployment element 102, and a second deployment element 104. Each of the first deployment element 102 and second deployment element 104 is configured to hold a corresponding portion of an expandable heart valve prosthesis in a radially collapsed condition.

In embodiments, the first deployment element 102 may be provided as a cup member featuring an ogee-like shape, preferably with a blunt end to avoid damaging to the patient's tissues and vasculature.

The first deployment element 102 of the carrier portion P100 is connected to a drive feature enabling it to slide along the axis X100 and operable through a drive member preferably located on the handle itself. The drive member may include a knob for operation by the practitioner.

Connection to the drive feature may occur for example via a connecting rod or shaft member 106 that is slidably arranged into the shaft of the delivery instrument 100.

The second deployment element 104 may be - instead - provided as a sheath member including a plug 108 and a sheath 109 fitted onto the plug 108 (e.g. by interference fitting, or else thermally bonded thereto). The connecting rod or shaft member 106 may run through the plug 108 and the hub 101, and be slidable relative thereto. The hub 101 may advantageously comprise radial guide blades 110 for the sheath 109, and a locking feature 112, particularly in the form of a locking flange or collar, configured to receive and lock a corresponding portion of the armature 2 of the valve prosthesis 1.

The plug 108 is fixed to the handle via a drive feature enabling it to slide along the axis X100 and operable through a drive member preferably located on the handle itself. The drive member may include a knob for operation by the practitioner. In embodiments, each of the deployment elements 102, 104 may be independently operable of each other, for example via individually operable drive members, so to independently control the releasing action provided thereby. In other embodiments, both deployment elements may be operatively connected to respective drive features, and be nevertheless operable via a single and common drive member. An example of such embodiments is disclosed in PCT application no. PCT/IB2018/053646 in the name of the same Applicant.

Whatever the drive arrangement, the deployment instrument 100 is operable to delivery and release a heart valve prosthesis - such as the prosthesis 1-including a radially contractible/radially expandable armature and a prosthetic valve carried thereby to an implantation site.

For this purpose, the deployment elements 102, 104 of the carrier portion P100 are each operable from a respective first axial position wherein an overlap occurs with a respective portion of the heart valve prosthesis 1 loaded on the instrument 100 - particularly on the hub 101 of the carrier portion P100 - to hold the same portion in a radially collapsed condition, to a second position wherein no overlap occurs between the deployment element 102, 104 and the prosthesis 1, which is thus in a radially expanded condition.

In embodiments featuring a drive arrangement with a common drive for both the deployment elements 102, 104, these deployment elements are operable from a position of minimum (allowed) mutual distance condition, corresponding to both deployment elements 102, 104 in the first operating position and associated to a loading/delivery operating condition of the prosthesis 1, to a maximum (allowed) mutual distance condition corresponding to both deployment elements 102, 104 in the second operating position and associated to complete valve deployment/release at the implantation site. The distance referred to is that along the axis X100.

In the embodiments referred to in figures 31 and 32, the first deployment element 102 is associated to the inflow portion IF of the prosthesis 1, while the second deployment element 104 is associated to the outflow portion OF of the heart valve prosthesis. In this latter regard, the ends 20 are received and locked onto the locking feature 112, with the sheath 109 in overlapping condition when in the first position.

In embodiments herein, with reference to figure 31, the kit may include the delivery instrument 100 with the prosthesis 1 pre-mounted/pre-loaded thereon with the outflow portion OF held in a radially contracted condition by the deployment element 104 in the respective first position, and the inflow portion IF held in a radially contracted condition by the deployment element 102 in the respective first position. The anchoring formations 6 are not subject to overlap by the deployment elements 102, 104, and as such are not subject to a direct radial contraction. An example of such a loading pattern is disclosed in European Patent no. EP 2 238 947 B1 in the name of the same applicant, figure 3B thereof.

The pre-loading arrangement of figure 31 is applicable both in case of independently operable deployment elements 102, 104 and in case of commonly driven deployment elements 102, 104.

The assembled kit may then be sterilized - for example by ethylene oxide - and packed for storage prior to use by the practitioner. In this way, the practitioner will be relieved from all of the crimping and loading operations as the kit will actually provide a ready to use facility. The "dry" implantable material ID exhibits excellent dry storage properties due, i.a., to the presence of polyols (such as glycerol). The implantable material remains resilient and supple even after months of dry storage, and as such opens to the possibility of a joint storage with the delivery instrument 100.

In other embodiments, with reference to figure 32, the kit may include the delivery instrument 100 with the prosthesis 1 pre-mounted/pre-loaded thereon with the outflow portion OF held in a radially contracted condition by the deployment element 104 in the respective first position, and the inflow portion IF free of radial contraction by the deployment element 102, which is accordingly in the respective second position. This arrangement may be applicable with independently operable deployment elements 102, 104 and may be intended for long-term storage to minimize distortions of the leaflets 4 - provided that the same will be kept stored in a collapsed condition until the actual use of the kit.

Accordingly, at the time of use the practitioner will be required to collapse the inflow portion IF to complete loading of the prosthesis 1 onto the carrier portion. Radial collapse of the inflow portion may be provided, e.g., by a crimping tool which may be possibly provided as part of the kit.

Naturally, while the principle of the invention remains the same, the details of construction and the embodiments may widely vary with respect to what has been described and illustrated purely by way of example, without departing from the scope of the present invention.

## Claims

1. A method of providing features (LCD, HD, HD_IN, HD_OUT) on an implantable material (ID), the method comprising:
- providing an implantable material (ID) comprising a decellularized sterilized mammalian tissue having an extracellular matrix, wherein a plurality of interstitial spaces of the extracellular matrix include a solution of one or more polyols,
- providing one or more features (LCD, HD, HD_IN, HD_OUT) on said implantable material (ID) by laser treatment.

2. The method of Claim 1, wherein said laser treatment includes laser cutting.

3. The method according to Claim 1 or Claim 2, wherein said laser treatment includes CO₂ laser treatment.

4. The method according to Claim 1 or Claim 2, wherein said laser treatment includes femtosecond laser treatment.

5. The method according to any of Claims 2 to 4, wherein said providing one or more features (LCD, HD, HD_IN, HD_OUT) on said implantable material (ID) by laser treatment includes laser cutting a line feature (LCD), and wherein said laser cutting a line feature (LCD) includes laser cutting a perimeter and/or an edge of an element out of said implantable material (ID).

6. The method according to Claim 5, wherein said laser cutting a line feature includes laser cutting a closed loop feature (HD), and wherein said laser cutting a closed loop feature includes laser cutting one or more through holes (HD).

7. The method according to any of Claims 2 to 4, wherein said providing one or more features (4) on said implantable material (ID) by laser treatment includes laser ablation to provide a sculpturing of said implantable material (ID).

8. The method according to Claim 1, wherein the implantable material is dried and sterilized, and wherein the solution of one or more polyols includes one or more polyols other than glycerol, glycerol.

9. The method according to Claim 8, wherein the glycerol is present in an amount of about 1 vol. % to about 50 vol. % of the mixture and the one or more polyols other than glycerol are present in an amount of about 50 vol. % to about 99 vol. % of the mixture.

10. The method according to Claim 8, wherein the one or more polyols other than glycerol includes at least one of 1,2 propanediol, 1,2 octanediol, 1,4 butanediol, fructose, xylitol, mannitol, sorbitol, lactulose, lactic acid, maltose, glucose, galactose, erythritol, lactobionic acid or its salts, and hyaluronic acid.

11. The method according to Claim 8, wherein the one or more polyols other than glycerol are selected from the group consisting of 1,2 propanediol, 1,4 butanediol, lactobionic acid and its salts, and combinations thereof.

12. An implantable cardiovascular prosthesis having at least a portion made of an implantable material having features provided by the method according to any of Claims 1 to 11.

13. The implantable cardiovascular prosthesis of Claim 12, wherein the prosthesis is a heart valve prosthesis (1) including a plurality of prosthetic valve leaflets (4) made of said implantable material (ID).

14. The implantable cardiovascular prosthesis (1) of Claim 13, wherein each leaflet (4) features laser cut edges, and one or more laser cut through holes (SH) providing sewing locations.

15. The implantable cardiovascular prosthesis (1) of any of Claims 13, 14, wherein the prosthetic leaflets (4) include sculptured features (CP, DP), said sculptured features including at least one of:
- a contoured sculptured pattern (CP),
- a deformation pattern (DP)
- a layer ablation.

16. A method of making an implantable cardiovascular prosthesis (1) according to any of claims 12 to 15, the method including:
- providing a sheet of said implantable material (ID) comprising a decellularized sterilized mammalian tissue having an extracellular matrix, wherein a plurality of interstitial spaces of the extracellular matrix include a solution of one or more polyols,
- laser cutting a plurality of through line features into said implantable material (ID) to define corresponding perimeters (PM) of a plurality of leaflets (4)
- laser cutting a plurality of through closed loop features into said implantable material (ID) to provide a plurality of sewing holes (SH) for each leaflet (4),
- sewing the leaflets (4) into a valvular sleeve in correspondence of said sewing holes (SH), and
- sewing the leaflets (4) to a supporting structure (2) of said implantable cardiovascular prosthesis (1).

17. The method of Claim 16, further including providing a sculpturing by laser ablation within the perimeter (OM) of each leaflet (4).

18. A kit comprising a delivery instrument (100) and an implantable cardiovascular prosthesis (1) according to any of claims 12 to 15.
